# EUROPEAN PATENT SPECIFICATION

(11) **EP 1 845 903 B1**
(45) Date of publication and mention of the grant of the patent: **10.04.2013**
(21) Application number: 05857774.3
(22) Date of filing: 14.12.2005
(51) Int. Cl.: A61F 2/44, A61F 2/30, A61F 2/00

(54) **INTERVERTEBRAL DISC PROSTHESIS**
BANDSCHEIBENPROTHESE
PROTHESE DE DISQUE INTERVERTEBRAL

(30) Priority: 22.12.2004 FR 0413728
(43) Date of publication of application: 24.10.2007
(60) Divisional application: 11165170.9
(73) Proprietor: LDR Medical, 10430 Rosières près Troyes (FR)
(72) Inventor: ZEEGERS, Willem, NL-6231 EJ Meerssen (NL)
(74) Representative: Debay, Yves
(86) International application number: PCT/IB2005/004093
(87) International publication number: WO 2006/120505

(56) References cited:
- WO-A-02/089701
- WO-A-2004/034935
- WO-A-2004/089256
- DE-A1- 10 323 363
- DE-U1- 20 320 454
- FR-A- 2 846 550

## Description

The present invention relates to an intervertebral disc prosthesis, intended to be substituted for fibro-cartilaginous discs ensuring a bond between the vertebrae of the spinal column.

Various types of intervertebral disc prostheses are known in the prior art. Numerous prostheses, such as for example in the patent application WO 02 089 701 and WO 2004/041129, are constituted in a lower plate and an upper plate forming a sort of cage around a central core. A part of these prostheses enables the upper plate to swivel in relation to the central core and optionally permits the central core to slide in relation to the lower plate. This sliding of the central core in relation to the lower plate is an essential characteristic, as it must allow spontaneous positioning of the core in the ideal position to absorb constraints imposed on the prosthesis, during movements made by the patient wearing the prosthesis. The displacement of the core, co-operating with at least a plate about an uneven surface, enables an inclination between the plates of the prosthesis which facilitates the mobility of the patient wearing the prosthesis. The displacement of the core also prevents it from creeping under load, when subjected to major constraints. A part of these prostheses have osseous anchorage means allowing to attach these prostheses to the vertebrae between which they are intended to be inserted.

However, the size of the vertebrae varies greatly from person to person, for a same vertebra in a given position in the spinal column, but also for a given person depending on the position of the vertebrae in the spinal column between which a prosthesis is intended to be inserted. The intervertebral disc prostheses must be of a suitable size for the vertebrae between which they are intended to be inserted, depending on the person and on the position of these vertebrae in the spinal column. Moreover, depending on the spinal column disorder of the patient wearing the prosthesis, it is sometimes preferable that the prosthesis allows a correction of this disorder. The prostheses can thus be used to correct an inclination defect of the vertebrae, such as, for example, lordosis. It is known from prior art, in particular from Patent Applications WO 2004/034935 and DE 203 20 454 comprising at least two plates and at least one core, wherein the size of the plate can vary to be adapted to the size of the vertebrae between which the prosthesis is intended to be implanted. Patent Applications WO 2004/034935 also describes convex surfaces, chamfers or rounding on the surface of the plates contacting the vertebrae, for a good contact between the prosthesis and the surfaces of the vertebral bodies. To have prostheses that are suitable for the as big a majority of cases as possible, a large number of prostheses with different plate sizes and inclinations must therefore be envisaged. This multiplicity of prostheses has the major inconvenience of high manufacturing costs and high stock levels. In this context, it is beneficial to propose a prosthesis that has a structure allowing it to be adapted to different sizes of vertebrae and allowing for different inclinations of the plates. Such a prosthesis would reduce stock levels and manufacturing costs.

The aim of the present invention is to propose an intervertebral disc prosthesis allowing limited movements of the different pieces of the prosthesis between one another and comprising a core used to restrict its displacement in at least one direction. A same prosthesis can be adapted to different sizes of vertebrae at reduced costs.

This aim is achieved by an intervertebral disc prosthesis comprising at least three pieces including an upper plate, a lower plate, and a movable core at least in relation to a plate, having the characterising features of claim 1.

According to another feature, the anatomic adaptation elements consist in crowns which surround the plates and prolong respectively their upper and lower surfaces to present contact surfaces of the prosthesis with the adjacent vertebrae which are bigger than when there are no anatomic adaptation elements.

According to another feature, the anatomic adaptation elements symmetrically prolong the upper and lower surfaces of respectively the upper and lower plates to present an equivalent prolongation of these surfaces on the different anterior, posterior and lateral edges of the plates.

According to another feature, the anatomic adaptation elements asymmetrically prolong the upper and lower surfaces of respectively the upper and lower plates to present a bigger prolongation of these surfaces on at least one of the anterior, posterior and lateral edges of the plates than on the other edges.

According to another feature, the upper surface of the core is in contact with at least one part of the lower surface of the upper plate and the lower surface of the core is in contact with at least one part of the upper surface of the lower plate.

According to another feature, at least one part of the surface of at least a plate is concave and complementary with a convex surface of the core with which it is in contact.

According to another feature, at least one part of the surface of at least a plate is plane and complementary with a plane surface of the core with which it is in contact.

According to another feature, male and female co-operation means situated in the vicinity of the edges of at least one plate and the core limiting, without excessive friction, the movements in translation of the core relative to this plate, according to an axis substantially parallel to this plate, and limiting or suppressing the movements in rotation of the core relative to this plate, about an axis substantially perpendicular to this plate.

According to another feature, the dimensions of each male co-operation means are slightly less than those of each female co-operation means so as to allow slight clearance between the core and the plate equipped with these co-operation means.

According to another feature, the dimensions of each male co-operation means are substantially the same as those of each female co-operation means so as to prevent any clearance between the core and the plate equipped with these co-operation means.

According to another feature, the co-operation means of the plate are female co-operation means co-operating with male co-operation means of the core.

According to another feature, the male co-operation means of the core are two blocks situated on the two side edges of the core and the female co-operation means of the plate are four walls situated, in pairs, on each of the two side edges of this plate.

According to another feature, the fixation means of the anatomic adaptation elements on the plates of the prosthesis are reversible and allow changing the anatomic adaptation elements fixed in a movable manner onto the plates of the prosthesis.

According to another feature, the fixation means of the anatomic adaptation elements on the plates consist in fixation means present on the anatomic adaptation elements and complementary with fixation means present on the plates of the prosthesis.

According to another feature, the anatomic adaptation elements are fixed onto the plates via, on one hand, contact with at least a part of their surfaces which face at least a part of the plates and, on the other hand, contact of their fixation means with the complementary fixation means present on the plates of the prosthesis.

According to another feature, the fixation means of the anatomic adaptation elements on the plates consist in male fixation means present on the anatomic adaptation elements and co-operating with the female fixation means present on the plates of the prosthesis or inversely.

According to another feature, the female fixation means present on the plates of the prosthesis consist in plane surfaces present on the edges of the plates of the prosthesis.

According to another feature, the female fixation means present on the plates of the prosthesis consist in recesses made in the edges of the other plate of the prosthesis.

According to another feature, the female fixation means present on the plates of the prosthesis consist in recesses made in the edges of the female co-operation means of the plates of the prosthesis.

According to another feature, the female fixation means present on the plates of the prosthesis consist in plane surfaces present on the edges of one of the plates and in recesses made in the female co-operation means of the edges of the other plate of the prosthesis.

According to another feature, the female fixation means present on at least one of the plates of the prosthesis consist in plane surfaces present on at least a first edge of one of the plates and in recesses made in at least a second edge of the plate of the prosthesis, the second edge geometrically facing a first edge of the plate.

According to another feature, at least one of the female fixation means present on the plates of the prosthesis comprises at least a notch allowing blocking the male fixation means of the anatomic adaptation elements on this female fixation means.

According to another feature, the fixation means of the anatomic adaptation elements on the plates consist in female fixation means present on the anatomic adaptation elements and co-operating with male intermediary means which can also co-operate with the female fixation means present on the plates of the prosthesis.

According to another feature, the anatomic adaptation elements are fixed onto the plates via, on one hand, contact of at least a part of their upper and lower surface with at least a part of respectively the upper and lower plates and, on the other hand, contact of the male intermediary means with the female fixation means present on the anatomic adaptation elements and with the female fixation means present on the plates of the prosthesis.

According to another feature, the male intermediary means possess securing means blocking the male intermediary means in the position where they co-operate with both the female fixation means of the anatomic adaptation elements and with the female fixation means present on the plates of the prosthesis.

According to another feature, the male intermediary means consist in a sliding plate in the female fixation means present on the anatomic adaptation elements to co-operate with the female fixation means present on the plates of the prosthesis, the securing means of the male intermediary means consisting in at least a formal irregularity present on at least one side of this plate and intended to co-operate with at least an opening made in the female fixation means of the anatomic adaptation elements and/or in the female fixation means of the plates, thus blocking the male intermediary means in the position where they co-operate with both the female fixation means of the anatomic adaptation elements and with the female fixation means present on the plates of the prosthesis.

According to another feature, the securing means of the male intermediary means consist in a bore in the male intermediary means and in the female fixation means present on the anatomic adaptation elements, the bore in the female fixation means of the anatomic adaptation elements being intended to receive a securing pin blocking the male intermediary means in the position where they co-operate with the female fixation means present on the plates of the prosthesis.

According to another feature, the median planes representing the upper and lower surfaces of each of the anatomic adaptation elements are substantially parallel or form an acute angle, the inclination obtained by such an angle allowing to adapt the overall shape of the prosthesis to the anatomy of the spinal column or to possibly correct inclination defects of the vertebrae of the patient for whom the prosthesis is intended.

According to another feature, the same anatomic adaptation elements are assembled with different plates whose upper and lower surfaces create different angles.

According to another feature, an angle between the upper surface of the upper plate and the lower surface of the lower plate is imposed either by the fact that the median planes representing the upper and lower surfaces of the lower plate and/or the upper plate create an angle, or by restricting, thanks to the co-operation means, movements of the core about a position imposing an inclination of at least one of the plates.

According to another feature, the same plates are assembled with cores of different thicknesses and/or sizes and/or shapes.

According to another feature, the anatomic adaptation elements comprise movable osseous anchorage elements that are fixed onto the anatomic adaptation elements upon fixing the anatomic adaptation elements onto the plates, inserting the prosthesis between the vertebrae and possibly adjusting the relative position of the different elements of the prosthesis.

According to another feature, the movable osseous anchorage elements of the anatomic adaptation elements consist in at least a plate equipped with notches oriented to resist against the removal of the plate once it has been inserted into a vertebra, a far end of the plate bearing a part curved to fold over itself and intended to be interlocked as a hook onto an edge of an opening made in the vicinity of the periphery of the anatomic adaptation elements.

According to another feature, the part, curved to fold over itself, of the notched plate of the movable osseous anchorage means of the anatomic adaptation elements prolongs with a second plate also equipped with notches oriented to resist against the removal of the plate once it has been inserted into the vertebra.

According to another feature, the anatomic adaptation elements comprise movable osseous anchorage elements consisting in at least one winglet to be inserted in a groove performed in the adjacent surfaces of the vertebrae between which the prosthesis is to be implanted, said winglet comprising notches oriented to resist against the ejection of the prosthesis outside its housing between the vertebrae, a far end of the winglet bearing a part curved to fold over itself and intended to be interlocked as a hook onto an edge of an opening made in the vicinity of the periphery of the anatomic adaptation elements.

According to another feature, the winglet further comprises a pin having dimensions adapted so that it tightly fits into a groove of the anatomic adaptation elements and/or the plates.

Other features and advantages of the invention will emerge more clearly from the description herein below, given in reference to the attached diagrams, in which:
- figure 1 illustrates an exploded perspective view of the different elements of the prosthesis according to an embodiment of the invention,
- figure 2 illustrates an exploded perspective view of the different elements of the prosthesis according to another embodiment of the invention,
- figure 3 illustrates a perspective view of the prosthesis according to another embodiment of the invention.
- figures 4A and 4B respectively illustrate a bottom view and a cross section view along plane A-A in figure 4A, of the upper plate equipped with its anatomic adaptation element, according to an embodiment of the invention, figures 4C and 4D respectively illustrate a plan view and a cross section view along plane B-B in figure 4C, of the upper plate equipped with its anatomic adaptation element, according to an embodiment of the invention,
- figure 5A illustrates a bottom view of the upper plate equipped with its anatomic adaptation element, according to an embodiment of the invention, and figures 5B and 5C illustrate cross section views respectively along plane C-C and plane D-D in figure 5A, of the upper plate equipped with its anatomic adaptation element, according to this embodiment of the invention,
- figures 6A and 6B illustrate bottom views of a part of the upper plate equipped with its anatomic adaptation element, according to two different embodiments of the invention,
- figures 7A and 7B illustrate perspective views of the lower plate equipped with its anatomic adaptation element, according to two different embodiments of the invention,
- figures 8A and 8B respectively illustrate a bottom view and a cross section view along plane E-E in figure 8A, of a part of the lower plate equipped with its anatomic adaptation element whose fixation means are open, according to an embodiment of the invention, figures 8C and 8D respectively illustrate a bottom view and a cross section view along plane F-F in figure 8C, of the same embodiment as in figures 8A and 8B, but with the fixation means of the anatomic adaptation element closed and locked, according to an embodiment of the invention,
- figures 9A and 9B respectively illustrate a bottom view and a cross section view along plane G-G in figure 9A, of a part of the lower plate equipped with its anatomic adaptation element whose fixation means are open, according to an embodiment of the invention, figures 9C and 9D respectively illustrate a bottom view and a cross section view along plane H-H in figure 9C, of the same embodiment as in figures 9A and 9B, but with the fixation means of the anatomic adaptation element closed and locked, according to an embodiment of the invention,
- figures 10A and 10B illustrate perspective views of, respectively, the prosthesis comprising osseous anchorage means according to an embodiment of the present invention and one of the osseous anchorage means according this embodiment,
- figures 11A and 11B respectively illustrate a perspective view of the prosthesis comprising osseous anchorage means according to an embodiment of the present invention and a cross section view along plane I-I of figure 11A.

The intervertebral disc prosthesis according to the present invention is constituted in an upper plate (1) articulated in relation to a lower plate (2) by means of a core (3) and each of the plates (1, 2) is equipped with an anatomic adaptation element (11, 22) allowing to adjust the overall size of the prosthesis to the size of the vertebrae between which the prosthesis is intended to be inserted. Thus, thanks to the anatomic adaptation elements (11, 22), a single unit constitutes two plates (1, 2) and the core (3) can be used for different sizes of vertebrae, which has the advantage of substantially reducing the cost of manufacturing prostheses and their variances. The advantage of the prosthesis according to the invention is that it comprises simple parts whose anatomic adaptation elements (11, 22) can be sized so as to adapt to different vertebrae of the spinal column, for example, the adjust the thickness of the prosthesis to the intervertebral gap and/or adjust the inclination of the plates (1, 2) of the prosthesis to the inclination of the vertebrae of the patient. Even though the anatomic adaptation elements (11, 22) allow themselves to adjust the prosthesis to different sizes of vertebrae , the plates (1, 2) and the core (3) of differing sizes and shapes can naturally be used if needs be.

The two anatomic adaptation elements (11, 22) of the prosthesis according to the invention consist in an upper element (11) and a lower element (12). The upper element (11) has, on one hand, an upper surface (110) of which at least a part presents a surface in contact with a lower surface of a first vertebra and has, on the other hand, a lower surface (111) of which at least a part presents a surface in contact with a part of the upper plate (1). The lower element (22) has, on one hand, a lower surface (220) of which at least a part presents a surface in contact with an upper surface of a second vertebra and has, on the other hand, an upper surface (222) of which at least a part presents a surface in contact with a part of the lower plate (2). Each of the two anatomic adaptation elements (11, 22) is fixed onto the plates (1, 2) via respective fixation means (113, 223).

The core (3) is of slight thickness (from 3 to 15 mm, depending on the vertebrae between which the prosthesis is to be inserted). For good absorption of the constraints, the core (3) could, for example, be made of polyethylene, a compressible material simulating the physical properties of elasticity of natural intervertebral discs.

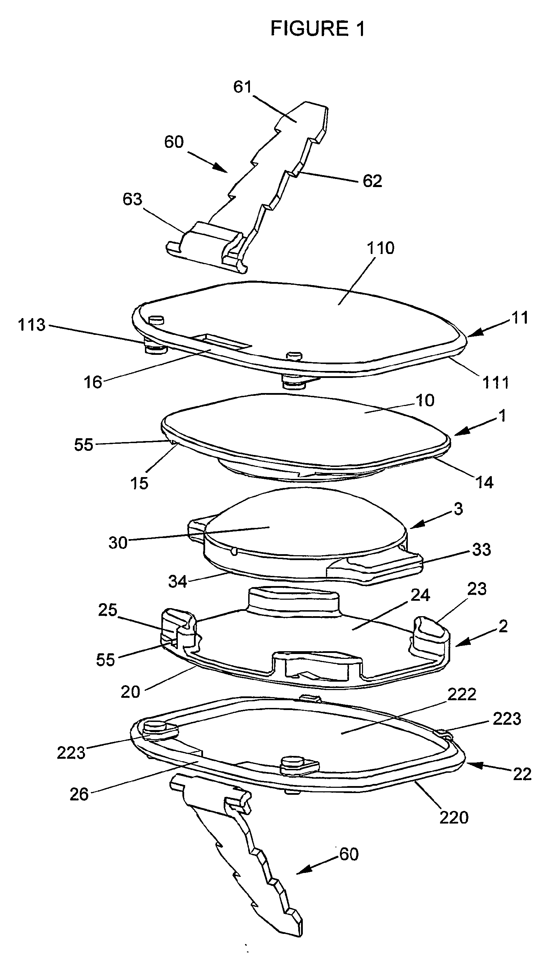

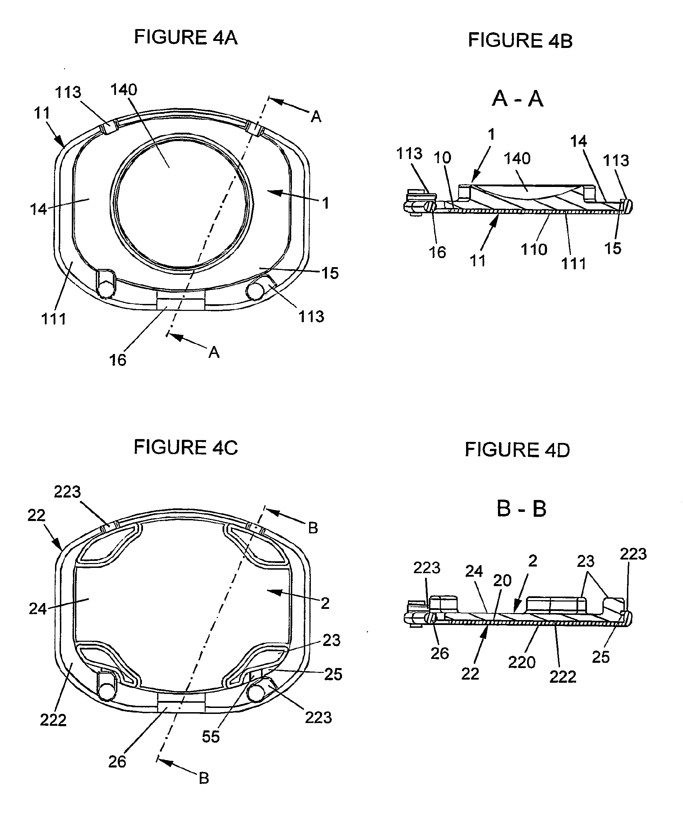

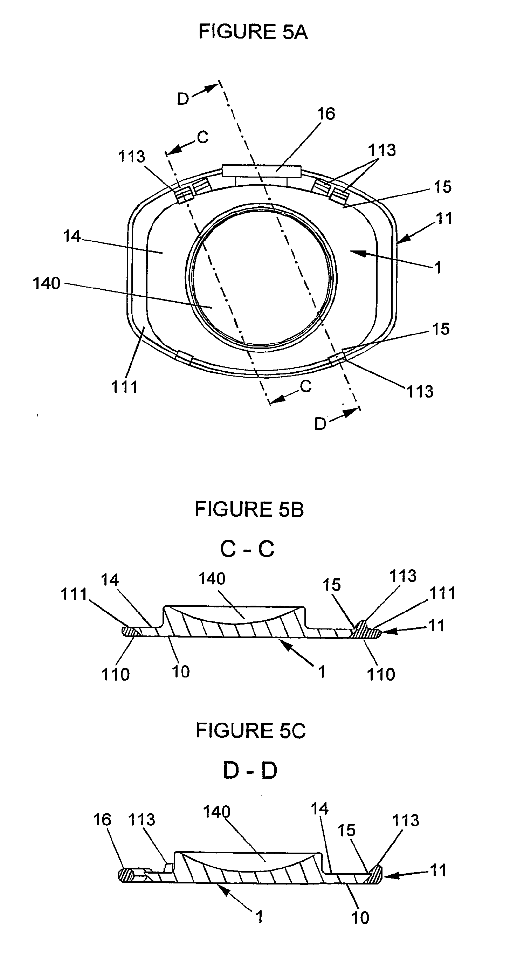

In all the possible embodiments of the invention, the core (3) has a convex part on at least a part of at least one of its upper (30) and lower (34) surfaces. In the embodiments illustrated in figures 1 to 9, it is the upper surface (30) of the core (3) which is convex and complementary with a concave part (140) of the lower surface (14) of the upper plate (1), whereas the lower surface (34) of the core (3) is plane and complementary with at least a plane part of the upper surface (24) of the lower plate (2). The concave part (140) of the lower surface (14) of the upper plate (1), as particularly visible in figures 4A, 4B, 5A, 5B and 5C, has a circular periphery. In other possible embodiments (not shown), it is a part of the lower surface (34) of the core (3) that can be convex and complementary with a concave part of the upper surface (24) of the lower plate (2), whereas the upper surface (30) of the core (3) is plane and complementary with at least a plane part of the lower surface (14) of the upper plate (1). In other embodiments (not shown), the concave surface lies on a part of one of the upper (30) and lower (34) surfaces of the core (3) and co-operates with a convex surface which lies on a part of a surface of one of the plates (1, 2). In these possible different embodiments (not shown) of the invention, the non convex or non concave surface of the core (3) can respectively be concave or convex, for example very slightly.

In the embodiments illustrated in figures 1 to 9, the concave part (140) of the lower surface (14) of the upper plate (1) complementary with the convex part of the upper surface (34) of the core (3) allows to incline the upper plate (1) when the patient wearing the prosthesis bends over. The co-operation between the concave surface (140) and the convex surface (34) presents a surface of articulation with the prosthesis, thanks to this inclination of the upper plate (1) in relation to the core (3). The centre of this articulation is naturally at the tip of the convex surface (34) of the core (3). In the illustrated embodiments, the lower surface of the core (3) and the upper surface of the lower plate (2) are plane so as to permit clearance of the core (3) in relation to the lower plate (2), both in translation according to an axis substantially parallel to the lower plate (2), and in rotation about an axis substantially perpendicular to the lower plate (2). During movements by the patient wearing the prosthesis, this inclination of the upper plate (1) and this clearance of the core will allow displacement of the core (3) towards the ideal position to absorb the constraints applied to the prosthesis. The movement between the upper plate (1) and the core (3), as well as the clearance of the core (3) in relation to the lower plate (2) thus allow the patient to move, and, optionally, to eliminate the defects of positioning the prosthesis. This clearance likewise has the advantage of preventing premature wear due to the constraints applied to the prosthesis.

Irrespective of the embodiment, the core (3) also has male or female co-operation means (33) complementary with respectively female or male co-operation means (23) present on at least one of the plates (1, 2). These male and female co-operation means (23, 33) situated in the vicinity of the edges of at least one plate (1, 2) and of the core (3) limit, without excessive friction, the movements in translation of the core (3) in relation to this plate (1, 2), according to an axis substantially parallel to this plate (1, 2), and limiting or suppressing the movements in rotation of the core (3) in relation to this plate (1, 2), about an axis substantially perpendicular to this plate (1, 2). The dimensions of each male co-operation means (33) can be slightly less than those of each female co-operation means (23) so as to allow slight clearance between the core (3) and the plate (1, 2) equipped with these co-operation means. On the contrary, the dimensions of each male cooperation means (33) can also be substantially the same as those of each female co-operation means (23) so as to prevent any clearance between the core (3) and the plate (1, 2) equipped with these co-operation means.

In the embodiment in figures 1 to 3, the core (3) has male co-operation means (33) complementary with female co-operation means (23) present on the lower plate (2). The male co-operation means (33) of the core (3) are, for example, hasps or blocks substantially parallelepiped in shape, present on the side edges of the core (3), as particularly visible in figures 1 to 3. The female co-operation means (23) can consist, for example, in four walls situated, in pairs, on each of the two side edges of the lower plate (2). These walls could be curved to the centre of the prosthesis, so as to cover at least a part of the male co-operation means (33) of the core (3) and avoid lifting the core (3) and the upper plate (1). These co-operation means (23, 33) also prevent the core (3) from ejecting out of the prosthesis, in the event of too much constraint on the prosthesis. In an alternative embodiment (not shown), the dimensions of each male co-operation means (33) of the core (3) are substantially the same as those of each female co-operation means (23) of the lower plate (2), so as to avoid any clearance of the core (3) in relation to the lower plate (2), both in translation and in rotation. In the latter case, the only permitted movement of the prosthesis is the inclining of the upper plate (1) in relation to the core (3). In an alternative embodiment (not shown), the core (3) has female co-operation means, consisting, for example, in complementary recesses of the male means present on the lower plate (2). These male means of the lower plate (2) can consist, for example, in two blocks or two nibs, for example curved to the interior of the prosthesis and facing one another on two edges of the lower plate (2). The nibs can, for example, be replaced by a block with a bore on which is fixed a hasp by way of a pin penetrating the bore. In another alternative embodiment (not shown), the lower plate (2) has half dog points. The core (3), by way of complement, has wells under its lower surface. The dimensions of the half dog points of the lower plate (2) and of the wells of the core (3) will be adapted, by choice, by a slight clearance of the core (3) in translation and in rotation or by no clearance, according to the desired result. In other alternatives (not shown), the co-operation means can be located on the core (3) and on the upper plate (1), instead of the lower plate (2).

The description of a first embodiment will now be considered in reference to figure 1. In this embodiment, the upper (11) and lower (12) anatomic adaptation elements consist in plates, called anatomic, which respectively cover the upper (1) and lower (2) plates. The upper (222) and lower (111) surfaces of respectively the lower (22) and upper (11) anatomic adaptation elements can have a reinforcement in which respectively the lower (2) and upper (1) plates are housed. In another alternative, these upper (222) and lower (111) surfaces of the anatomic adaptation elements can be plane and comprise stoppers which, as for the aforementioned reinforcements, prevent respectively the lower (2) and upper (1) plates from moving in relation to the anatomic adaptation elements. The upper (222) and lower (111) surfaces of respectively the lower (22) and upper (11) anatomic adaptation elements prolong respectively the upper (10) and lower (20) surfaces of the upper (1) and lower (2) plates, to present contact surfaces of the prosthesis with the adjacent vertebrae which are bigger than when there are no anatomic adaptation elements (11, 22). Different sizes of the anatomic plates of the anatomic adaptation elements (11, 22) can be adapted to a single unit created by the two plates (1, 2) and the core (3), to provide good contact between the prosthesis and the vertebrae of differing sizes.

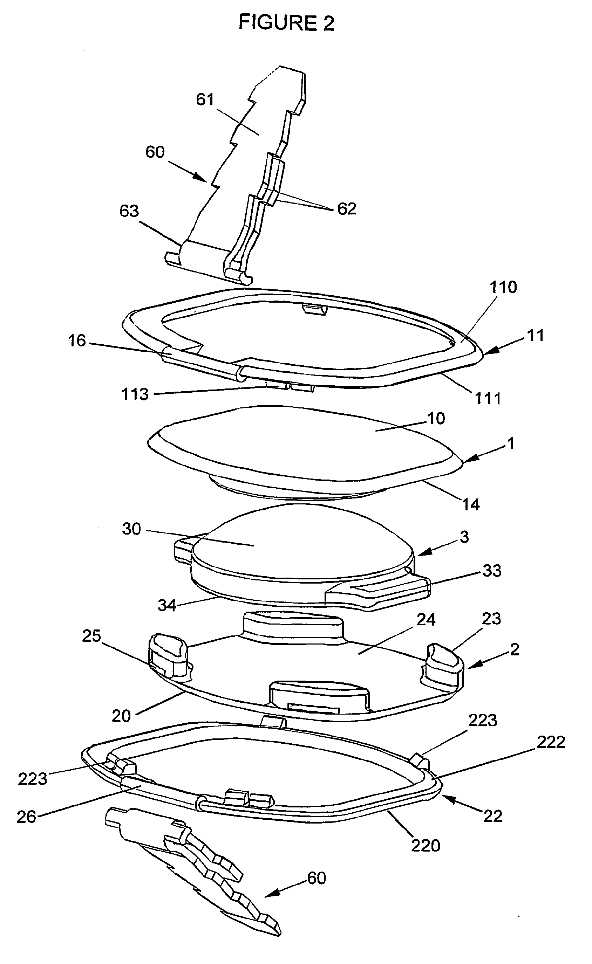

In the embodiment of the prosthesis according to the invention illustrated in figure 2, the anatomic adaptation elements (11, 22) consist in crowns which surround the upper (1) and lower (2) plates. In this embodiment, the edges of the upper (10) and lower (20) surfaces of respectively the upper (1) and lower (2) plates are bevelled and complementary with respectively the lower (111) and upper (222) inside edges of respectively the upper (11) and lower (22) crowns. This inclined shape of the edges of the plates (1, 2) and of the anatomic adaptation crowns (11, 22) co-operates with the fixation means (113, 223) of the anatomic adaptation elements in order to maintain the anatomic adaptation crowns (11, 22) fixed in plane of respectively the upper (1) and lower (2) plates of the prosthesis. The anatomic adaptation crowns (11, 22) prolong respectively the upper (10) and lower (20) surfaces of respectively the upper (1) and lower (2) plates, to present contact surfaces of the prosthesis with the adjacent vertebrae which are bigger than when there are no anatomic adaptation elements (11, 22). In the same manner as for the aforementioned anatomic plates (11, 22), a single unit created by the two plates (1, 2) and the core (3) can thus be adjusted to vertebrae of differing sizes, thanks to different sizes of crowns of the anatomic adaptation elements (11, 22).

In all the embodiments of the invention, the anatomic adaptation elements (11, 22) can symmetrically or asymmetrically prolong the upper (10) and lower (20) surfaces of respectively the upper (1) and lower (2) plates. Thus, for example, the anterior edge of the anatomic adaptation elements (11, 22) can have a bigger contact surface with the vertebrae than its posterior edge, so that the centre of articulation of the prosthesis (described above) is centred in relation to the natural axis of the spinal column, that meaning off centre to the rear of the vertebrae of a 2/3-1/3 section.

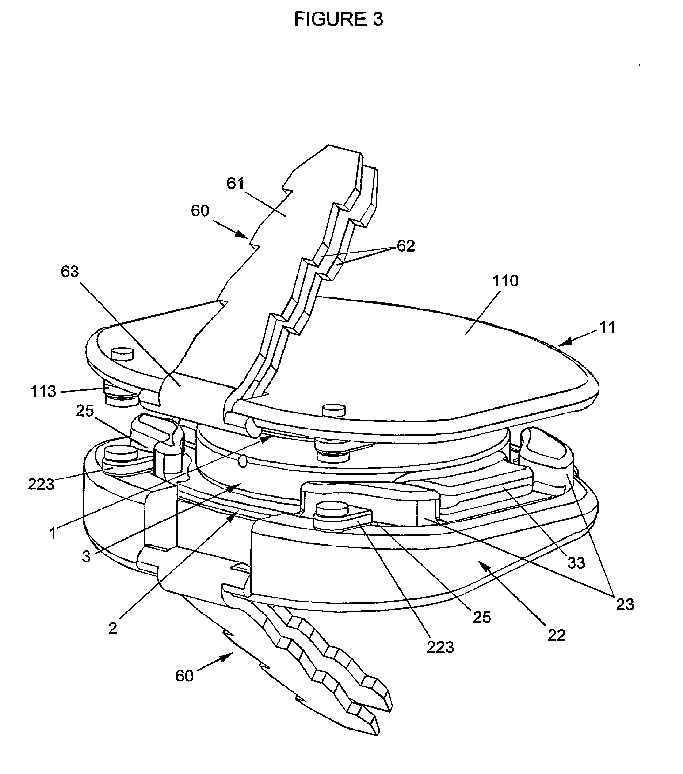

According to the chosen embodiments, the intervertebral disc prosthesis according to the invention allows, for example, to correct the defects of lordosis. The presence of an angle between the upper and lower surfaces of the prosthesis, in contact with the adjacent vertebrae, could be desirable. Such an angle could be obtained by making an upper plate (1), whose median planes representing its lower (14) and upper (10) surfaces create an angle. Another possibility consists in that it is the lower plate (2) whose median planes representing its lower (20) and upper (24) surfaces create an angle. Another possibility consists in that it is at least one of the anatomic adaptation elements (11, 22) whose median planes representing its lower and upper surfaces create an angle. Thus, a single unit made of the two plates (1, 2) and the core (3) can be used, for example, to induce or not lordosis, depending on which anatomic adaptation elements (11, 22) are associated to it. In the embodiment illustrated in figure 3, the lower surface (220) of the lower anatomic plate (22) creates an angle with its upper surface (222). Another possibility to obtain such an angle consists in a slightly offset position of the core (3) in relation to the centre of the prosthesis. This slightly offset position of the core (3) can, for example, be maintained thanks to an adjustable positioning of the male and female co-operation means (23, 33) between themselves. If the surgeon wishes, for example, that the prosthesis induces lordosis which remains within a range of values, he will select a prosthesis whose core (3) can have slight clearance in translation and in rotation in relation to the lower plate (2), but about a position imposing a slight permanent inclination of at least one of the plates, thanks to an accurate adjustment of the co-operation means (23, 33) between the core (3) and the lower plate (2). Thus, according to the chosen embodiment, the median planes representing the upper (110, 222) and lower (111, 220) surfaces of each of the anatomic adaptation elements (11, 22) can be substantially parallel or form an acute angle. The inclination obtained by such an angle allows to adapt the overall shape of the prosthesis to the anatomy of the spinal column or to possibly correct inclination defects of the vertebrae of the patient for whom the prosthesis is intended. The same anatomic adaptation elements (11, 22) can be assembled with different plates (1, 2) whose upper (10, 24) and lower (14, 20) surfaces create different angles. On the contrary, the plates (1, 2), whose upper (10, 24) and lower (14, 20) surfaces are parallel, are assembled with the anatomic adaptation elements (11, 22) whose upper (110, 222) and lower (111, 220) surfaces create different angles. This angle between the upper (10) surface of the upper plate (1) and the lower surface (20) of the lower plate (2) can be imposed either by the fact that the median planes representing the lower (20, 14) and upper (24, 10) surfaces of the lower plate (2) and/or the upper plate (1) create an angle, or by restricting, thanks to the co-operation means (23, 33), movements of the core (3) about a position imposing an inclination of at least one of the plates (1, 2).

Illustrated in figures 1 to 3 are the movable osseous anchorage means (60) of the anatomic adaptation elements (11, 22). Advantageously, these osseous anchorage means (60) can be fixed onto the anatomic adaptation elements (11, 22) upon fixing them onto the plates (1, 2) and most of all upon inserting the prosthesis between the vertebrae. This feature allows the surgeon to easily position the prosthesis between the vertebrae and then insert the osseous anchorage means (60) once the prosthesis has been correctly positioned. In the embodiment presented in figure 1, these movable osseous anchorage means (60) consist in a plate (61) equipped with notches (62) oriented to resist against the removal of the plate (61) once it has been inserted into the vertebra. This plate (61) can, of course, be replaced by a rod in the shape of a nail, for example, with or without notches (62) to resist against its removal from the vertebra. A far end of the plate (61) bears a part (63) curved to fold over itself. This curved part forms a kind of a hook intended to be interlocked onto an edge (16, 26) of an opening made in the vicinity of the periphery of the anatomic adaptation elements (11, 22). This edge (16, 26) of the opening creates a sort of rod onto which the osseous anchorage means (60) interlock. Indeed, the curved part (63) allows clasping the osseous anchorage means (60) onto the rod (16, 26) of the anatomic adaptation elements (11, 22). This rod can, of course, be replaced by any equivalent means allowing to clasp the osseous anchorage means (60). In the embodiments illustrated in figures 1 to 9, the rod (16, 26) is located on the anterior edge of the anatomic adaptation elements (11, 22) so as to allow the surgeon access once the prosthesis has been inserted between the vertebrae via anterior means (through accessing the vertebrae from their anterior face). If the implanting of the prosthesis is to be done via posterior means, the anatomic adaptation elements (11, 22) could have a rod (16, 26) located on the posterior edge. If the implanting of the prosthesis is to be done via lateral means, the anatomic adaptation elements (11, 22) could have a rod (16, 26) located on at least one of their edges. In the embodiment illustrated in figures 2 and 3, the hooked part (63), curved to fold over itself, of the notched plate (61) of the movable osseous anchorage means (60) of the anatomic adaptation elements (11, 22) prolong with a second plate (61) also equipped with notches (62) oriented to resist against the removal of the plate (61) once it has been inserted into the vertebra. In the embodiment illustrated in figure 2, this second plate (61) is shorter than the first plate and in the embodiment illustrated in figure 3, it is as long as the first plate. The fact that the osseous anchorage means (60) locks onto the rod (16, 26) allows it to have a variable angle which facilitates the attaching of the prosthesis. Indeed, depending on its encumbrance, the surgeon will have a choice of angles according to which he wishes to drive the osseous anchorage means (60) into the vertebrae. Moreover, the fact that the osseous anchorage means (60) can be inserted after positioning the prosthesis between the vertebrae this allows to adjust the relative position of the different elements (1, 2, 3) of the prosthesis. Indeed, the inserting of the prosthesis generates constraints on the elements of the prosthesis which are movable in relation to each other and they risk being badly position together. The surgeon can then, thanks to the invention, adjust the position of the prosthesis between the vertebrae and adjust the relative position of the elements of the prosthesis between themselves prior to definitively attaching the prosthesis.

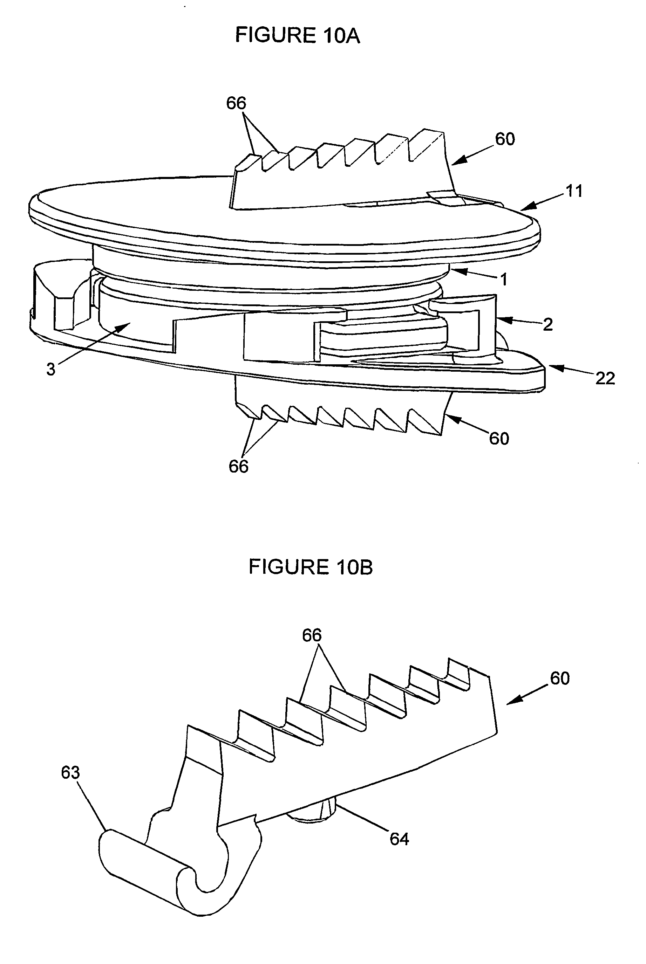

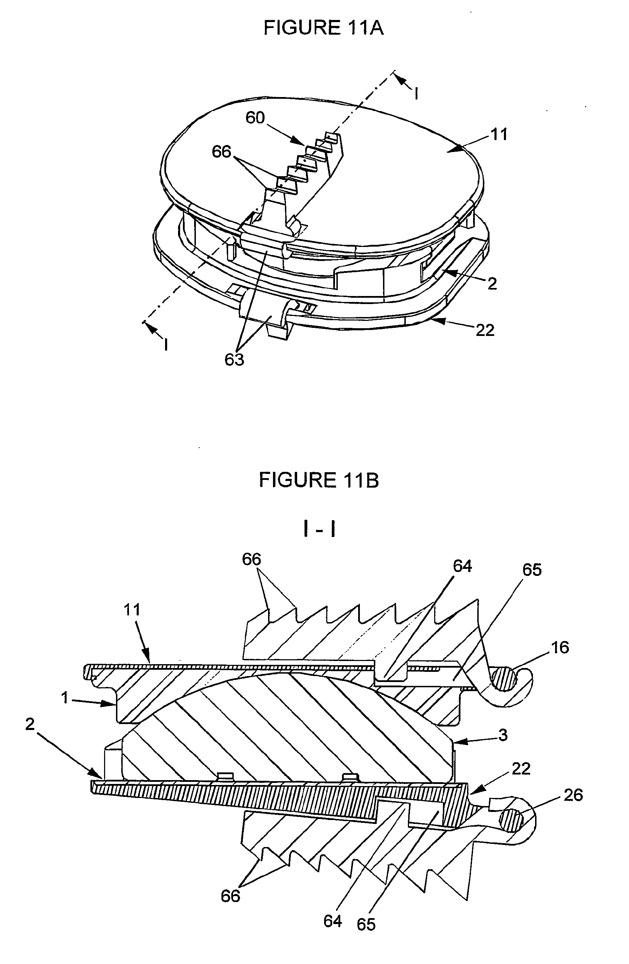

It is obvious that the present prosthesis can comprise other osseous anchorage means (60) than those described above, without departing from the scope of the present invention. To give non-limitative examples, such osseous anchorage means (60) can consist of winglets fixed on the prosthesis as in the Patent Application WO03/039400 or of a stud nailed in the vertebra through the anatomic adaptation elements as in the Patent Application WO04/041129. One embodiment of the anchorage means (60) is presented on the figures 10A, 10B, 11A and 11B. The osseous anchorage means (60) according to this embodiment consist of winglets comprising a hooked part (63), curved to fold over itself, so that the winglets can be adapted onto the anatomic adaptation elements. The hooked part (63) of the winglet, particularly visible on figure 10B, allows the anchorage means (60) to be interlocked onto the edge (16, 26) of an opening made in the vicinity of the periphery of the anatomic adaptation elements (11, 22), as particularly visible on figures 11A and 11B. This edge (16, 26) of the opening creates a sort of rod onto which the osseous anchorage means (60) interlock, as described above. The winglet further comprises a pin (64) (or a dowel) adapted to be inserted into a groove (65) present on the surface of the plate and/or the anatomic adaptation element on which the winglet is to be fixed, as particularly visible on figure 11 B. The groove (65) and the pin (64) have dimensions adapted so that the pin (64) is secured into groove (65). For example, the pin (64) may have a substantially conical shape, with the larger diameter of the cone being at the base of the pin and the smaller diameter being at its end. The groove (65) may have its side walls adapted to cooperate with the conical shape of the pin (64) so that the pin tightly fits inside the groove and thus secures the osseous anchoring means (60) onto the anatomic adaptation elements (11, 22). For example, the width of the groove (65) may be larger at its surface than at its bottom. The osseous anchorage means (60) are thus fixed onto the prosthesis of the present invention by first interlocking the hooked part (63) onto the rod (16, 26) of the anatomic adaptation elements (11, 22) and by rotating the osseous anchorage means (60) around the rod until the pin (64) penetrates tightly into the groove (65) of the anatomic adaptation element (11, 22) and/or of the plate (1, 2). The winglet (60) can have a standard size for all the prosthesis made according to the present invention and the position of the pin (64) of the winglet (60) inside the groove (65) will vary as a function of the size of the anatomic adaptation elements (11, 22). Depending on the thickness of the anatomic adaptation elements (11, 22), the pin (64) penetrates into the anatomic adaptation element (11, 22) only or may traverse the anatomic adaptation element (11, 22) and penetrate into a groove (65) in the plates (1, 2), as shown for example on figure 11B for the upper plate (1). Since the anatomic adaptation element (11, 22) vary in size (diameter), their groove may have variable lengths and can be replaced by a hole having a variable distance from the rod (16, 26) so that the hole is adapted to receive the pin (64), but when the pin is designed to penetrate the plates also, the plates will have to include a groove because the distance of the pin from the periphery of the plates will vary depending on the size of the anatomic adaptation element (11, 22). Once secured onto the anatomic adaptation elements (11, 22), the winglets (60) are adapted to cooperate with a groove drilled in the surfaces of the adjacent vertebrae with which they are in contact. Thus, the surgeon may perform a groove in the surfaces of vertebrae between which the prosthesis is intended to be inserted. This groove in the vertebrae will naturally have an orientation relative to the sagittal plane that will depend on the position and orientation of the winglet. This orientation will be predetermined and will set and secure the orientation of the prosthesis. Similarly, the depth of the groove in the vertebrae and its extend from the periphery will be predetermined as a function of the size of the winglet (60) and will allow the surgeon to adjust the relative position of the various elements of the prosthesis and to predict the position of the prosthesis relative to the natural axis of the vertebrae. The winglets comprise notches (66) on their surfaces which are intended to be in contact with the bottom of the groove performed in the vertebrae. These notches (66) of the winglets (60) will resist against the ejection of the prosthesis from inside its housing between the vertebrae, for example when strong constraints are applied to the prosthesis. It is obvious (particularly from the figure 11 B showing both embodiments) that the hooked part (63) of the winglets (60) can be oriented so that they are to be interlocked onto the rod (16, 26) by being inserted inside the opening made in the vicinity of the periphery of the anatomic adaptation elements (11, 22) or by being inserted from outside this opening.

It is obvious that the osseous anchoring means (60) described above are particularly adapted to the anatomic adaptation elements (11, 22) of the present invention but may also be adapted to the plates of other types of intervertebral disc prosthesis having plates comprising an opening in the vicinity of their periphery. The edge (16, 26) of such opening in the plates create a sort of rod (16, 26) onto which the hooked part (63) of both removable embodiments of the osseous anchoring means (60) can be interlocked.

Figures 4 to 9 illustrate the plates (1, 2) of the prosthesis traverse equipped with their anatomic adaptation elements (11, 22) and define different embodiments of the fixation means (113, 223, 15, 25) of these anatomic adaptation elements (11, 22) on the plates (1, 2). These fixation means (113, 223, 15, 25) are reversible, this means that the anatomic adaptation elements (11, 22) can be easily attached and removed from the plates (1, 2) of the prosthesis. These fixation means (113, 223, 15, 25) allow the anatomic adaptation elements (11, 22), fixed in a moveable manner to the plates (1, 2), to be changed. The fixation means (113, 223, 15, 25) of the anatomic adaptation elements (11, 22) on the plates (1, 2) consist in fixation means (113, 223) present on the anatomic adaptation elements (11, 22) and complementary with the fixation means (15, 25) present on the plates (1, 2) of the prosthesis. The anatomic adaptation elements (11, 22) are fixed onto the plates (1, 2) via, on one hand, contact of at least a part of their lower (111) and upper (222) surface with at least a part of respectively the upper (1) and lower (2) plates and, on the other hand, contact of their fixation means (113, 223) with the complementary fixation means (15, 25) present on the plates (1, 2) of the prosthesis. For the anatomic plates (11, 22) such as those illustrated, for example, in figures 4A to 4D, the anatomic adaptation elements (11, 22) are fixed onto the plates (1, 2) thanks to the fact that the upper (10) and lower (20) surfaces of respectively the upper (1) and lower (2) plates are firmly attached to the reinforcements present on the lower (111) and upper (222) surfaces of respectively the upper (11) and lower (22) anatomic plates, via the fixation means (113, 223, 15, 25). For the anatomic crowns (11, 22) such as those illustrated, for example, in figures 5A to 5C, the anatomic adaptation elements (11, 22) are fixed onto the plates (1, 2) thanks to the fact that the bevelled parts of the upper (10) and lower (20) surfaces of respectively the upper (1) and lower (2) plates are firmly attached to the bevelled parts of the upper (111) and lower (222) surfaces of respectively the upper (11) and lower (22) anatomic plates, via the fixation means (113, 223, 15, 25). The different embodiments of the fixation means (113, 223, 15, 25) of the anatomic adaptation elements (11, 22) on the plates (1, 2) will now be described in reference to figures 4 to 9. It is obvious that these fixation means are given by way of illustration and can be replaced by any equivalent means without leaving the scope of the invention. Likewise, the invention allows the use of any whatsoever combination of the different fixation means (113, 223, 15, 25) described below.

In several embodiments, the fixation means (113, 223, 15, 25) of the anatomic adaptation elements (11, 22) on the plates (1, 2) consist in male fixation means (113, 223) present on the anatomic adaptation elements (11, 22) and co-operate with the female fixation means (15, 25) present on the plates (1, 2) of the prosthesis. The female fixation means (15, 25) present on the plates (1, 2) of the prosthesis can consist, for example, in plane surfaces (15, 25) present on the edges of the plates (1, 2) of the prosthesis or in recesses (15, 25) either made in the edges of the plates (1, 2) of the prosthesis, or in the edges of the female co-operation means (23) of the plates (1, 2) of the prosthesis.

In an embodiment illustrated in figures 4A and 4B, the fixation means (113) of the upper anatomic plate (11) consist, on the posterior edge of its lower surface (111), in nibs which are shaped and have dimensions intended to receive a section (15) of the posterior edge of the lower surface (14) of the upper plate (1). On the anterior edge of its lower surface (111), the fixation means (113) of the upper anatomic plate (11) consist in latches constituted in an axis of rotation onto which a hasp is mounted intended to swivel about this axis and receive a section (15) of the posterior edge of the lower surface (14) of the upper plate (1), as particularly visible in figures 4A and 4B. The right-hand latches in figures 4A to 4C are illustrated in the open position and the left-hand latches are in the closed position. In the embodiment illustrated in figures 4C and 4D, the fixation means (223) of the lower anatomic plate (22) consist, on the posterior edge of its lower surface (222), in nibs which are shaped and have dimensions intended to fit into an opening (25) made in the co-operation means (23) of the lower plate (2). On the anterior edge of its lower surface (222), the fixation means (223) of the upper anatomic plate (22) consist in latches constituted in an axis of rotation onto which a hasp is mounted intended to receive a recess (25) made in a part of the co-operation means (23) present on the posterior edge of the lower plate (2). The latches illustrated in figures 4A to 4D can be maintained in the closed position via securing means (55) present, for example, on the plates (1, 2) of the prosthesis. For example, as illustrated in figure 4C, a notch (55) made on the recess (25) present on a part of the co-operation means (23) of the lower plate (2) prevents the latch (223) of the lower anatomic plate (22) from swivelling.

In the embodiment in figures 5A to 5C, the anterior and posterior edges of the upper anatomic adaptation crowns (11) have fixation means (113) consisting in nibs which co-operate with a plane section (15) present on the edge of the lower surface (14) of the upper plate.

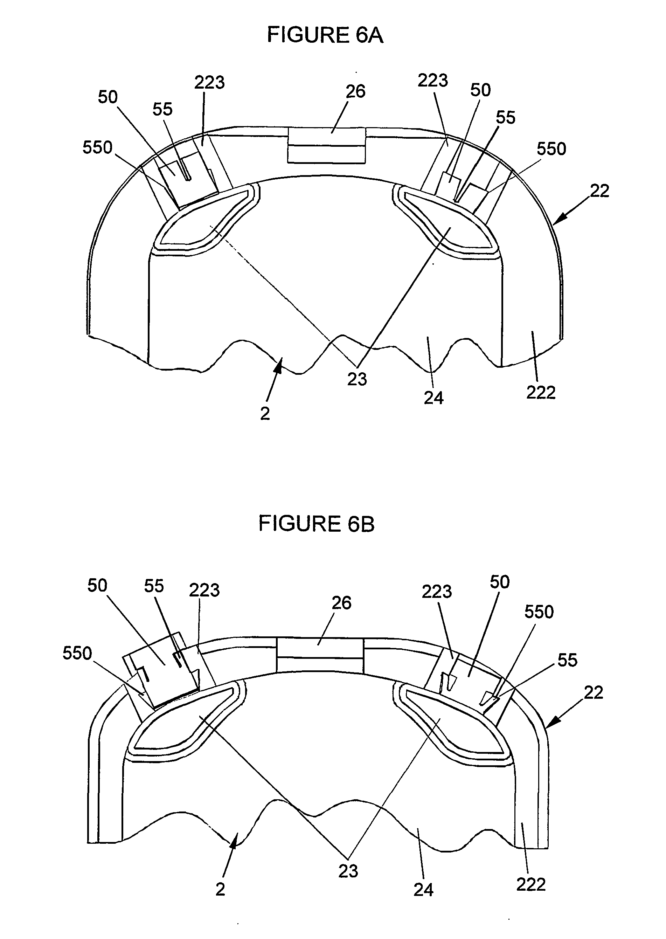

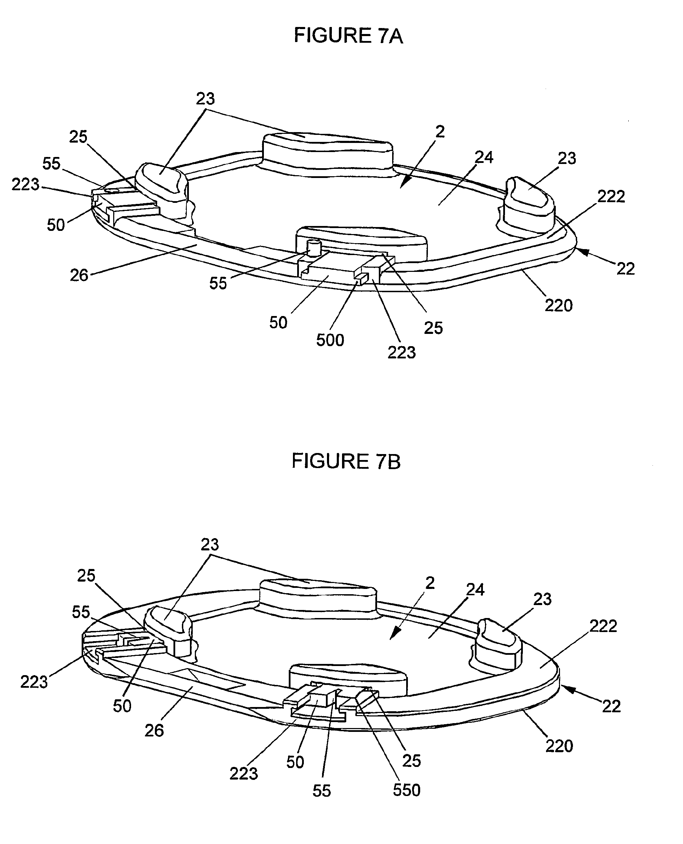

In the embodiments in figures 6A to 9D, the fixation means (113, 223, 15, 25) of the anatomic adaptation elements (11, 22) on the plates (1, 2) consist in female fixation means (113, 223) present on the anatomic adaptation elements (11, 22) and co-operating with male intermediary means (50) which can also co-operate with the female fixation means (15, 25) present on the plates (1, 2) of the prosthesis. The anatomic adaptation elements (11, 22) are fixed onto the plates (1, 2) via, on one hand, contact of at least a part of their upper (111) and lower (222) surface with at least a part of respectively the upper (1) and lower (2) plates and, on the other hand, contact of the male intermediary means (50) with the female fixation means (113, 223) present on the anatomic adaptation elements (11, 22) and with the female fixation means (15, 25) present on the plates (1, 2) of the prosthesis. The male intermediary means (50) consist in a sliding plate (50) in the female fixation means (113, 223) present on the anatomic adaptation elements (11, 22) to co-operate with the female fixation means (15, 25) present on the plates (1, 2) of the prosthesis. The plate (50) is substantially parallelepiped in shape and can comprise, on its side edges, fins (500), particularly visible, for example, in figure 7A. These fins (500) of the male intermediary means (50) are of complementary shape with the female fixation means (113, 223) of the anatomic adaptation elements (11, 22) and with the female fixation means (15, 25) of the plates (1, 2) of the prosthesis, which have side runners in which these fins (500) slide. This complementary shape of the fins (500) of the plate (50) and the runners of the female fixation means (113, 223) of the anatomic adaptation elements (11, 22) as well as the female fixation means (15, 25) of the plates (1, 2) prevent the plate (50) from leaving these female fixation means (113, 223, 15, 25) prior to being blocked via the securing means (55).

The male intermediary means (50) have securing means (55) blocking the male intermediary means (50) in the position where they co-operate with both the female fixation means (113, 223) of the anatomic adaptation elements (11, 22) and with the female fixation means (15, 25) present on the plates (1, 2) of the prosthesis. These securing means (55) which consist, for example, in at least a formal irregularity (55) present on at least one side of this plate (50) and intended to co-operate with at least an opening (550) made in the female fixation means (113, 223) of the anatomic adaptation elements (11, 22) and/or in the female fixation means (15, 25) of the plates (1, 2). The opening (550) can be of a complementary shape of the male intermediary means (50) or of its securing means, as illustrated in figures 6A and 6B.

In the embodiment illustrated in figure 6A, the plate constituting the male intermediary means (50) widens out towards its posterior end and the formal irregularity constituting the securing means (55) consists in a slot on the posterior half of the plate (50). This slot (55) compresses the posterior end of the plate (50) when it is introduced into the female fixation means (113, 223) of the upper and/or lower anatomic adaptation elements (11, 22), as illustrated for the left-hand plate in figure 6A. When the plate (50) reaches its end of stroke in the runner created by the female means (113, 223) of the anatomic adaptation elements (11, 22) and the means (15, 25) of the plates (1, 2), that meaning when the it co-operates with these two female means at the same time, openings (550) made, for example, in the female means (113, 223) of the anatomic adaptation elements (11, 22) separate the plate (50) from its hold, as illustrated for the right-hand plate in figure 6A. Figure 7B illustrates a perspective view of this embodiment of the fixation means in which the plate (50) is intended to be held in the female manes (25) made in the co-operation means (23) of the lower plate (2). This figure also notably shows the fact that the reinforcement present, for example, on the lower anatomic plate (22) can be deeper than the thickness of the lower plate (2). Depending on the size of the co-operation means (23, 33) of the lower plate (2) of the core (3), the edges of this reinforcement will provide a periphery abutment possibly limiting the displacement of the core (3) in relation to the lower plate (2). In the embodiment illustrated in figure 6B, the formal irregularities constituting the securing means (55) of the male intermediary means (50) consist in hasps present on the side edges of the plate (50). As illustrated for the left-hand plate (50) in figure 6B, these hasps (55) are compressed when the hasp is introduced into the runners of the female means (113, 223). When the plate is pushed as far as the blocking position, the hasps (55) naturally open out in the openings (550) provided for on the side edges of the female means (113, 223) of the upper and/or lower anatomic adaptation elements (11, 22), as illustrated for the right-hand plate (50) in figure 6B.

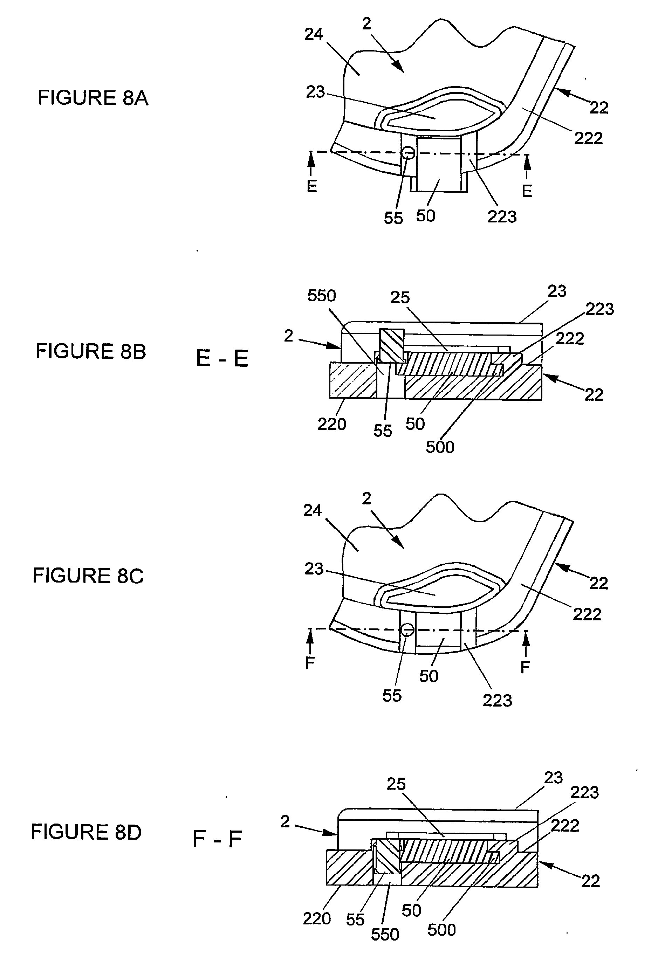

Figures 7A and 8A to 8D illustrate another alternative embodiment of the male intermediary means (50). In this embodiment, the formal irregularities of the plate (50) constituting the securing means (55) of the plate (50) consist in a bore in the male intermediary means (50), prolonged by a bore (550) in the female fixation means (113, 223) of the anatomic adaptation elements (11, 22), as particularly visible in figure 8B. The bore (550) is intended to receive a securing pin (55) blocking the male intermediary means (50) in the position where they co-operate with the female fixation means (15, 25) present on the plates (1, 2) of the prosthesis, as illustrated in figure 8C.

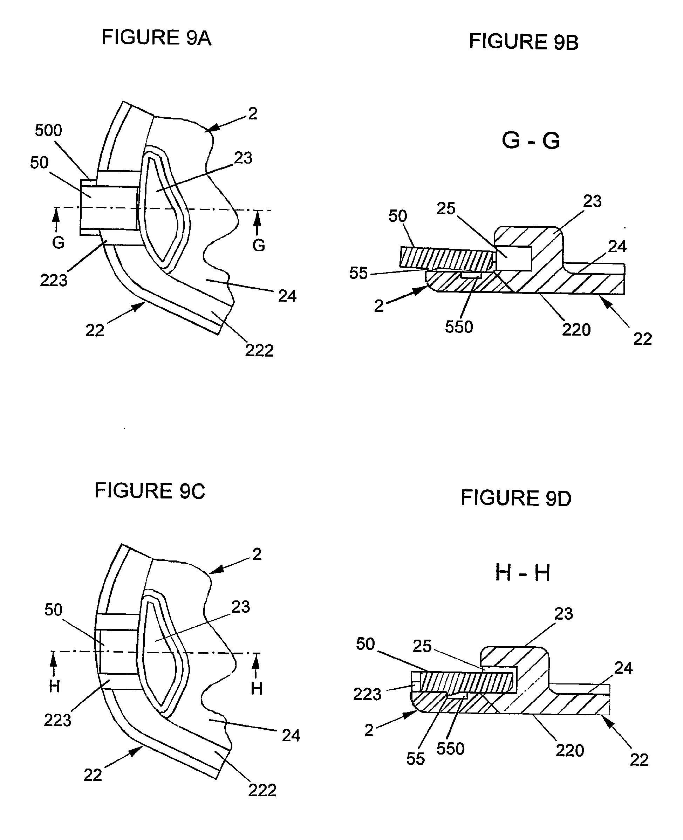

Another alternative of the securing means (55) of the male intermediary means (50) is illustrated in figures 9A to 9D. In this alternative, the formal irregularities constituting the securing means (55) of the plate (50) consist in a notch (55) present on the lower surface of the plate and intended to co-operate with an opening (550) made in the female fixation means (113, 223) of the anatomic adaptation elements by resisting against the removal of the plate (50), once driven as far as the female fixation means (15, 25) of the plates (1, 2), as illustrated in figure 9D.

It must be evident for specialists that the invention allows embodiments in numerous other specific forms without departing from the scope of application of the invention as claimed. As a consequence, the embodiments must be considered by way of illustration, but can be modified within the scope defined by the range of the attached claims, and the invention does not have to be limited to the details given above.

## Claims

1. An intervertebral disc prosthesis comprising a unit of a given size including at least three pieces including an upper plate (1), a lower plate (2), and a core (3) which is movable at least in relation to a plate, **characterised in that** it further comprises two anatomic adaptation elements (11, 22) each fixed onto one of the upper and lower plates (1, 2) via fixation means (113, 223) and allowing to adjust the overall size of the prosthesis, each adaptation element (11, 22) having, on one hand, a surface (110, 220) in contact with a surface of a vertebra and, on the other hand, a surface (111) of which at least a part has a surface in contact with at least a part of the plate (1, 2) onto which the anatomic adaptation element (11, 22) is mounted, anatomic adaptation elements (11, 22) of various sizes being fixed onto said unit of a given size in order to adapt the prosthesis to different sizes of vertebrae and/or different intervertebral gaps.

2. Intervertebral disc prosthesis set forth in claim 1, **characterised in that** the anatomic adaptation elements (11, 22) consist in crowns which surround the plates (1 and 2) and prolong respectively their upper (10) and lower (20) surfaces to present contact surfaces of the prosthesis with the adjacent vertebrae which are bigger than when there are no anatomic adaptation elements (11, 22).

3. Intervertebral disc prosthesis set forth in claim 1, **characterised in that** the anatomic adaptation elements (11, 22) consist in plates, called anatomic, which cover the plates (1 and 2) and prolong respectively their upper (10) and lower (20) surfaces to present contact surfaces of the prosthesis with the adjacent vertebrae which are bigger than when there are no anatomic adaptation elements (11, 22).

4. Intervertebral disc prosthesis set forth in any one of claims 1 to 3, **characterised in that** the anatomic adaptation elements (11, 22) symmetrically prolong the upper (10) and lower (20) surfaces of respectively the upper (1) and lower (2) plates to present an equivalent prolongation of these surfaces (10, 20) on the different anterior, posterior and lateral edges of the plates (1, 2).

5. Intervertebral disc prosthesis set forth in any one of claims 1 to 3, **characterised in that** the anatomic adaptation elements (11, 22) asymmetrically prolong the upper (10) and lower (20) surfaces of respectively the upper (1) and lower (2) plates to present a bigger prolongation of these surfaces (10, 20) on at least one of the anterior, posterior and lateral edges of the plates (1, 2) than on the other edges.

6. Intervertebral disc prosthesis set forth in any one of claims 1 to 5, **characterised in that** an upper surface (30) of the core (3) is in contact with at least one part of a lower surface (14) of the upper plate (1) and a lower surface (34) of the core (3) is in contact with at least one part of an upper surface (24) of the lower plate (2).

7. Intervertebral disc prosthesis set forth in any one of claims 1 to 6, **characterised in that** at least one part (140) of a surface (14, 24) of at least one plate (1, 2) is concave and complementary with a convex surface (30) of the core (3) with which it is in contact.

8. Intervertebral disc prosthesis set forth in any one of claims 1 to 7, **characterised in that** the at least one part of a surface (14, 24) of at least one plate (1, 2) is plane and complementary with a plane surface (34) of the core (3) with which it is in contact.

9. Intervertebral disc prosthesis set forth in any one of claims 1 to 8, **characterised in that** male and female co-operation means (23, 33) situated in the vicinity of the edges of at least one plate (1, 2) and the core (3) limiting the movements in translation of the core (3) relative to this plate (1, 2), according to an axis substantially parallel to this plate(1, 2), and limiting or suppressing the movements in rotation of the core (3) relative to this plate (1, 2), about an axis substantially perpendicular to this plate (1, 2).

10. Intervertebral disc prosthesis set forth in claim 9, **characterised in that** the dimensions of each male co-operation means (33) are slightly less than those of each female co-operation means (23) so as to allow slight clearance between the core (3) and the plate (1, 2) equipped with these co-operation means.

11. Intervertebral disc prosthesis set forth in claim 9, **characterised in that** the dimensions of each male co-operation means (33) are substantially the same as those of each female co-operation means (23) so as to prevent any clearance between the core (3) and the plate (1, 2) equipped with these co-operation means.

12. Intervertebral disc prosthesis set forth in any one of claims 9 to 11, **characterised in that** the co-operation means (23) of the plate (1, 2) are female co-operation means co-operating with male co-operation means (33) of the core (3).

13. Intervertebral disc prosthesis set forth in claim 12, **characterised in that** the male co-operation means (33) of the core (3) are two blocks situated on the two side edges of the core (3) and the female co-operation means (23) of the plate (1, 2) are four walls situated, in pairs, on each of the two side edges of this plate (1, 2).

14. Intervertebral disc prosthesis set forth in any one of claims 1 to 13, **characterised in that** the fixation means (113, 223, 15, 25) of the anatomic adaptation elements (11, 22) on the plates (1, 2) of the prosthesis are reversible and allow to change the anatomic adaptation elements (11, 22) fixed in a movable manner onto the plates (1, 2) of the prosthesis.

15. Intervertebral disc prosthesis set forth in any one of claims 1 to 14, **characterised in that** the fixation means (113, 223, 15, 25) of the anatomic adaptation elements (11, 22) on the plates (1, 2) consist in fixation means (113, 223) present on the anatomic adaptation elements (11, 22) and complementary with fixation means (15, 25) present on the plates (1, 2) of the prosthesis.

16. Intervertebral disc prosthesis set forth in claim 15, **characterised in that** the anatomic adaptation elements (11, 22) are fixed onto the plates (1, 2) via, on one hand, contact with at least a part of their surfaces (111 and 222) which face at least a part of the plates (1, 2) and, on the other hand, contact of their fixation means (113, 223) with the complementary fixation means (15, 25) present on the plates (1, 2) of the prosthesis.

17. Intervertebral disc prosthesis set forth in claim 15 or 16, **characterised in that** the fixation means (113, 223, 15, 25) of the anatomic adaptation elements (11, 22) on the plates (1, 2) consist in male fixation means (113, 223) present on the anatomic adaptation elements (11, 22) and co-operating with the female fixation means (15, 25) present on the plates (1, 2) of the prosthesis or inversely.

18. Intervertebral disc prosthesis set forth in claim 17, **characterised in that** the female fixation means (15, 25) present on the plates (1, 2) of the prosthesis consist in plane surfaces (15, 25) present on the edges of the plates (1, 2) of the prosthesis.

19. Intervertebral disc prosthesis set forth in claim 17, wherein the female fixation means (15, 25) present on the plates (1, 2) of the prosthesis consist in recesses (15, 25) made in the edges of the plates (1, 2) of the prosthesis.

20. Intervertebral disc prosthesis set forth in claim 17, **characterised in that** the female fixation means (15, 25) present on the plates (1, 2) of the prosthesis consist in recesses made in the edges of the female co-operation means (23) of the plates (1, 2) of the prosthesis.

21. Intervertebral disc prosthesis set forth in claim 17, **characterised in that** the female fixation means (15, 25) present on the plates (1, 2) of the prosthesis consist in plane surfaces (15) present on the edges of one of the plates (1) and in recesses (25) made in the female co-operation means (23) of the edges of the other plate (2) of the prosthesis.

22. Intervertebral disc prosthesis set forth in claim 17, **characterised in that** the female fixation means (15, 25) present on at least one of the plates (1, 2) of the prosthesis consist in plane surfaces (15, 25) present on at least a first edge of one of the plates (1, 2) and in recesses (15, 25) made in at least a second edge of the plate (1, 2) of the prosthesis, the second edge geometrically facing a first edge of the plate (1, 2).

23. Intervertebral disc prosthesis set forth in any one of claims 17 to 22, **characterised in that** at least one of the female fixation means (15, 25) present on the plates (1, 2) of the prosthesis comprises at least a notch (55) allowing to block the male fixation means (113, 223) of the anatomic adaptation elements (11, 22) on this female fixation means (15, 25).

24. Intervertebral disc prosthesis set forth in any one of claims 1 to 16, **characterised in that** the fixation means (113, 223, 15, 25) of the anatomic adaptation elements (11, 22) on the plates (1, 2) consist in female fixation means (113, 223) present on the anatomic adaptation elements (11, 22) and co-operating with male intermediary means (50) which also co-operates with the female fixation means (15, 25) present on the plates (1, 2) of the prosthesis.

25. Intervertebral disc prosthesis set forth in claim 24, **characterised in that** the anatomic adaptation elements (11, 22) are fixed onto the plates (1, 2) via, on one hand, contact of at least a part of their upper (111) and lower (222) surface with at least a part of respectively the upper (1) and lower (2) plates and, on the other hand, contact of the male intermediary means (50) with the female fixation means (113, 223) present on the anatomic adaptation elements (11, 22) and with the female fixation means (15, 25) present on the plates (1, 2) of the prosthesis.

26. Intervertebral disc prosthesis set forth in claim 24, **characterised in that** the male intermediary means (50) possess securing means (55) blocking the male intermediary means (50) in the position where they co-operate with both the female fixation means (113, 223) of the anatomic adaptation elements (11, 22) and with the female fixation means (15, 25) present on the plates (1, 2) of the prosthesis.

27. Intervertebral disc prosthesis set forth in claim 26, **characterised in that** the male intermediary means (50) consist in a sliding plate (50) in the female fixation means (113, 223) present on the anatomic adaptation elements (11, 22) to co-operate with the female fixation means (15, 25) present on the plates (1, 2) of the prosthesis, the securing means (55) of the male intermediary means (50) consisting in at least a formal irregularity (55) present on at least one side of this sliding plate (50) and intended to co-operate with at least an opening (550) made in the female fixation means (113, 223) of the anatomic adaptation elements (11, 22) and/or in the female fixation means (15, 25) of the plates (1, 2), thus blocking the male intermediary means formed by this sliding plate (50) in the position where they co-operate with both the female fixation means (113, 223) of the anatomic adaptation elements (11, 22) and with the female fixation means (15, 25) present on the plates (1, 2) of the prosthesis.

28. Intervertebral disc prosthesis set forth in claim 26, **characterised in that** the securing means (55) of the male intermediary means (50) consist in a bore in the male intermediary means (50) and in the female fixation means (113, 223) present on the anatomic adaptation elements (11, 22), the bore (550) in the female fixation means (113, 223) of the anatomic adaptation elements (11, 22) being intended to receive a securing pin (55) blocking the male intermediary means (50) in the position where they co-operate with the female fixation means (15, 25) present on the plates (1, 2) of the prosthesis.

29. Intervertebral disc prosthesis set forth in any one of claims 1 to 28, **characterised in that** the median planes representing the upper (110, 222) and lower (111, 220) surfaces of at least one of the anatomic adaptation elements (11, 22) are substantially parallel or form an acute angle, the inclination obtained by such an angle allowing to adapt the overall shape of the prosthesis to the anatomy of the spinal column or to possibly correct inclination defects of the vertebrae of the patient for whom the prosthesis is intended.

30. Intervertebral disc prosthesis set forth in any one of claims 1 to 29, **characterised in that** the same anatomic adaptation elements (11, 22) are assembled with different plates (1, 2) whose upper (10, 24) and lower (14, 20) surfaces create different angles.

31. Intervertebral disc prosthesis set forth in any one of claims 9 to 30, **characterised in that** an angle between the upper surface (10) of the upper plate (1) and the lower surface (20) of the lower plate (2) is imposed either by the fact that the median planes representing the lower (20, 14) and upper (24, 10) surfaces of the lower plate (2) and/or the upper plate (1) create an angle, or by restricting, thanks to the co-operation means (23, 33), movements of the core (3) about a position imposing an inclination of at least one of the plates (1, 2).

32. Intervertebral disc prosthesis set forth in any one of claims 1 to 31, **characterised in that** the same plates (1, 2) are assembled with cores (3) of different thicknesses and/or sizes and/or shapes.

33. Intervertebral disc prosthesis set forth in any one of claims 1 to 32, **characterised in that** the anatomic adaptation elements (11, 22) comprise movable osseous anchorage elements (60) that are fixed onto the anatomic adaptation elements (11, 22) upon fixing the anatomic adaptation elements (11, 22) onto the plates (1, 2), inserting the prosthesis between the vertebrae and possibly adjusting the relative position of the different elements (1, 2, 3) of the prosthesis.

34. Intervertebral disc prosthesis set forth in claim 33, **characterised in that** the movable osseous anchorage elements (60) of the anatomic adaptation elements (11, 22) consist in at least a plate (61) equipped with notches (62) oriented to resist against the removal of the plate (61) once it has been inserted into a vertebra, a far end of the plate (61) bearing a part (63) curved to fold over itself and intended to be interlocked as a hook onto an edge (16, 26) of an opening made in the vicinity of the periphery of the anatomic adaptation elements (11, 22).

35. Intervertebral disc prosthesis set forth in claim 34, **characterised in that** the part (63), curved to fold over itself, of the notched plate (61) of the movable osseous anchorage means (60) of the anatomic adaptation elements (11, 22) prolong with a second plate (61) also equipped with notches (62) oriented to resist against the removal of the plate (61) once it has been inserted into the vertebra.

36. Intervertebral disc prosthesis set forth in any one of claims 1 to 32, **characterised in that** the anatomic adaptation elements (11, 22) comprise movable osseous anchorage elements (60) consisting in at least one winglet to be inserted in a groove performed in the adjacent surfaces of the vertebrae between which the prosthesis is to be implanted, said winglet comprising notches (66) oriented to resist against the ejection of the prosthesis outside its housing between the vertebrae, a far end of the winglet (60) bearing a part (63) curved to fold over itself and intended to be interlocked as a hook onto an edge (16, 26) of an opening made in the vicinity of the periphery of the anatomic adaptation elements (11, 22).

37. Intervertebral disc prosthesis set forth in claim 36, **characterised in that** the winglet (60) further comprises a pin (64) having dimensions adapted so that it tightly fits into a groove (65) of the anatomic adaptation elements (11, 22) and/or the plates (1, 2).

## Patentansprüche

1. Bandscheibenprothese, die eine Einheit mit einer gegebenen Größe umfasst, die mindestens drei Teile aufweist, die eine obere Platte (1), eine untere Platte (2) und einen Kern (3), der mindestens in Bezug auf eine Platte beweglich ist, aufweist, **dadurch gekennzeichnet, dass** die Bandscheibenprothese weiterhin zwei anatomische Anpassungselemente (11, 22) umfasst, die jeweils mittels Befestigungsmitteln (113, 223) auf einer der oberen und der unteren Platte (1, 2) befestigt sind und ermöglichen, die Gesamtgröße der Prothese einzustellen, wobei jedes Anpassungselement (11, 22) einerseits eine Fläche (110, 220) in Kontakt mit einer Fläche eines Wirbel und andererseits eine Fläche (111) hat, von der mindestens ein Teil eine Fläche in Kontakt mit mindestens einem Teil der Platte (1, 2) hat, auf der das anatomische Anpassungselement (11, 22) montiert ist, wobei die anatomischen Anpassungselemente (11, 22) mit unterschiedlichen Größen auf der Einheit mit einer gegebenen Größe befestigt sind, um die Prothese auf unterschiedliche Größen von Wirbeln und/oder unterschiedliche Zwischenwirbelspalte anzupassen.

2. Bandscheibenprothese nach Anspruch 1, **dadurch gekennzeichnet, dass** die anatomischen Anpassungselemente (11, 22) aus Kronen bestehen, die die Platten (1 und 2) umschließen und deren obere (10) bzw. deren untere (20) Fläche verlängern, um Kontaktflächen der Prothese mit den angrenzenden Wirbeln zu bieten, die größer sind, als wenn es keine anatomischen Anpassungselemente (11, 22) gibt.

3. Bandscheibenprothese nach Anspruch 1, **dadurch gekennzeichnet, dass** die anatomischen Anpassungselemente (11, 22) aus Platten bestehen, die anatomische Platten genannt werden und die die Platten 1 und 2) bedecken und deren obere (10) bzw. deren untere (20) Fläche verlängern, um Kontaktflächen der Prothese mit den angrenzenden Wirbeln zu bieten, die größer sind, als wenn es keine anatomischen Anpassungselemente (11, 22) gibt.

4. Bandscheibenprothese nach einem der Ansprüche 1 bis 3, **dadurch gekennzeichnet, dass** die anatomischen Anpassungselemente (11, 22) die obere (10) und die untere (20) Fläche der oberen (1) bzw. der unteren (2) Platte symmetrisch verlängern, um eine äquivalente Verlägerung dieser Flächen (10, 20) an den unterschiedlichen vorderen, hinteren und seitlichen Kanten der Platten (1, 2) zu bieten.

5. Bandscheibenprothese nach einem der Ansprüche 1 bis 3, **dadurch gekennzeichnet, dass** die anatomischen Anpassungselemente (11, 22) die obere (10) und die untere (20) Fläche der oberen (1) bzw. der unteren (2) Platte asymmetrisch verlängern, um eine größere Verlängerung dieser Flächen (10, 20) an mindestens einer der vorderen, hinteren und seitlichen Kanten der Platten (1, 2) als an den anderen Kanten zu bieten.

6. Bandscheibenprothese nach einem der Ansprüche 1 bis 5, **dadurch gekennzeichnet, dass** eine obere Fläche (30) des Kerns (3) in Kontakt mit mindestens einem Teil einer unteren Fläche (14) der oberen Platte (1) ist und eine untere Fläche (34) des Kerns (3) in Kontakt mit mindestens einem Teil einer oberen Fläche (24) der unteren Platte (2) ist.

7. Bandscheibenprothese nach einem der Ansprüche 1 bis 6, **dadurch gekennzeichnet, dass** mindestens ein Teil (140) einer Fläche (14, 24) von mindestens einer Platte (1, 2) konkav ist und zu einer konvexen Fläche (30) des Kerns (3), mit der sie in Kontakt ist, komplementär ist.

8. Bandscheibenprothese nach einem der Ansprüche 1 bis 7, **dadurch gekennzeichnet, dass** der mindestens eine Teil einer Fläche (14, 24) von mindestens einer Platte (1, 2) eben ist und zu einer ebenen Fläche (34) des Kerns (3), mit der sie in Kontakt ist, komplementär ist.

9. Bandscheibenprothese nach einem der Ansprüche 1 bis 8, **dadurch gekennzeichnet, dass** männliche und weibliche Zusammenwirkungsmittel (23, 33), die sich in der Nähe der Kanten von mindestens einer Platte (1, 2) und des Kerns befinden, die Übertragungsbewegungen des Kerns (3) in Bezug auf diese Platte (1, 2) gemäß einer zu dieser Platte (1, 2) im Wesentlichen parallelen Achse beschränkt und die Drehbewegungen des Kerns (3) in Bezug auf diese Platte (1, 2) um eine zu dieser Platte (1, 2) im Wesentlichen senkrechten Achse beschränkt oder unterdrückt.

10. Bandscheibenprothese nach Anspruch 9, **dadurch gekennzeichnet, dass** die Abmessungen jedes männlichen Zusammenwirkungsmittels (33) geringfügig kleiner sind als die jedes weiblichen Zusammenwirkungsmittels (23), um einen geringen Abstand zwischen dem Kern (3) und der Platte (1, 2), die mit diesen Zusammenwirkungsmitteln ausgestattet sind, zu ermöglichen.

11. Bandscheibenprothese nach Anspruch 9, **dadurch gekennzeichnet, dass** die Abmessungen jedes männlichen Zusammenwirkungsmittels (33) zu denen jedes weiblichen Zusammenwirkungsmittels (23) im Wesentlichen gleich sind, um jeglichen Abstand zwischen dem Kern (3) und der Platte (1, 2), die mit diesen Zusammenwirkungsmitteln ausgestattet sind, zu verhindern.

12. Bandscheibenprothese nach einem der Ansprüche 9 bis 11, **dadurch gekennzeichnet, dass** die Zusammenwirkungsmittel (23) der Platte (1, 2) weibliche Zusammenwirkungsmittel sind, die mit männlichen Zusammenwirkungsmitteln (33) des Kerns zusammenwirken.

13. Bandscheibenprothese nach Anspruch 12, **dadurch gekennzeichnet, dass** die männlichen Zusammenwirkungsmittel (33) des Kerns (3) zwei Blöcke sind, die sich an zwei Seitenkanten des Kerns (3) befinden, und die weiblichen Zusammenwirkungsmittel (23) der Platte (1, 2) vier Wände sind, die sich paarweise an jeder der zwei Seitenkanten dieser Platte (1, 2) befinden.

14. Bandscheibenprothese nach einem der Ansprüche 1 bis 13, **dadurch gekennzeichnet, dass** die Befestigungsmittel (113, 223, 15, 25) der anatomischen Anpassungselemente (11, 22) auf den Platten (1, 2) der Prothese undrehbar sind und ermöglichen, die anatomischen Anpassungselemente (11, 22) zu ändern, die beweglich auf den Platten (1, 2) der Prothese befestigt sind.

15. Bandscheibenprothese nach einem der Ansprüche 1 bis 14, **dadurch gekennzeichnet, dass** die Befestigungsmittel (113, 223, 15, 25) der anatomischen Anpassungselemente (11, 22) auf den Platten (1, 2) aus Befestigungsmitteln (113, 223) bestehen, die auf den anatomischen Anpassungselementen (11, 22) vorliegen und zu Befestigungsmitteln (15, 25), die auf den Platten (1, 2) der Prothese vorliegen, komplementär sind.

16. Bandscheibenprothese nach Anspruch 15, **dadurch gekennzeichnet, dass** die anatomischen Anpassungselemente (11, 22) auf den Platten (1, 2) mittels einerseits Kontakt mit mindestens einem Teil ihrer Flächen (111 und 222), die mindestens einem Teil der Platten (1, 2) zugewandt sind, und andererseits Kontakt ihrer Befestigungsmittel (113, 223) mit den komplementären Befestigungsmitteln (15, 25), die auf den Platten (1, 2) der Prothese vorliegen, befestigt sind.

17. Bandscheibenprothese nach Anspruch 15 oder 16, **dadurch gekennzeichnet, dass** die Befestigungsmittel (113, 223, 15, 25) der anatomischen Anpassungselemente (11, 22) auf den Platten (1, 2) aus männlichen Befestigungsmitteln (113, 223) bestehen, die auf den anatomischen Anpassungselementen (11, 22) vorliegen und mit den weiblichen Befestigungsmitteln (15, 25), die auf den Platten (1, 2) der Prothese vorliegen, zusammenwirken oder umgekehrt.

18. Bandscheibenprothese nach Anspruch 17, **dadurch gekennzeichnet, dass** die weiblichen Befestigungsmittel (15, 25), die auf den Platten (1, 2) der Prothese vorliegen, aus ebenen Flächen (15, 25) bestehen, die auf den Kanten der Platten (1, 2) der Prothese vorliegen.

19. Bandscheibenprothese nach Anspruch 17, wobei die weiblichen Befestigungsmittel (15, 25), die auf den Platten (1, 2) der Prothese vorliegen, aus Aussparungen (15, 25) bestehen, die in den Kanten der Platten (1, 2) der Prothese hergestellt sind.

20. Bandscheibenprothese nach Anspruch 17, **dadurch gekennzeichnet, dass** die weiblichen Befestigungsmittel (15, 25), die auf den Platten (1, 2) der Prothese vorliegen, aus Aussparungen (15, 25) bestehen, die in den Kanten der weiblichen Zusammenwirkungsmittel (23) der Platten (1, 2) der Prothese hergestellt sind.

21. Bandscheibenprothese nach Anspruch 17, **dadurch gekennzeichnet, dass** die weiblichen Befestigungsmittel (15, 25), die auf den Platten (1, 2) der Prothese vorliegen, aus ebenen Flächen (15), die auf den Kanten einer der Platten (1) vorliegen, und aus Aussparungen (25) bestehen, die in den weiblichen Zusammenwirkungsmitteln (23) der Kanten der anderen Platte (2) der Prothese hergestellt sind.

22. Bandscheibenprothese nach Anspruch 17, **dadurch gekennzeichnet, dass** die weiblichen Befestigungsmittel (15, 25), die auf mindestens einer der Platten (1, 2) der Prothese vorliegen, aus ebenen Flächen (15, 25), die auf mindestens einer ersten Kante einer der Platten (1, 2) vorliegen, und aus Aussparungen (15, 25) bestehen, die in mindestens einer zweiten Kante der Platte (1, 2) der Prothese hergestellt sind, wobei die zweite Kante geometrisch einer ersten Kante der Platte (1, 2) zugewandt ist.

23. Bandscheibenprothese nach einem der Ansprüche 17 bis 22, **dadurch gekennzeichnet, dass** mindestens eines der weiblichen Befestigungsmittel (15, 25), die auf den Platten (1, 2) der Prothese vorliegen, mindestens eine Kerbe (55) umfasst, die ermöglicht, die männlichen Befestigungsmittel (113, 223) der anatomischen Anpassungselemente (11, 22) auf diesem weiblichen Befestigungsmittel (15, 25) zu arretieren.

24. Bandscheibenprothese nach einem der Ansprüche 1 bis 16, **dadurch gekennzeichnet, dass** die Befestigungsmittel (113, 223, 15, 25) der anatomischen Anpassungselemente (11, 22) auf den Platten (1, 2) aus weiblichen Befestigungsmitteln (113, 223) bestehen, die auf den anatomischen Anpassungselementen (11, 22) vorliegen und mit einem männlichen Zwischenmittel (50) zusammenwirken, das außerdem mit den weiblichen Befestigungsmitteln (15, 25) zusammenwirkt, die auf den Platten (1, 2) der Prothese vorliegen.

25. Bandscheibenprothese nach Anspruch 24, **dadurch gekennzeichnet, dass** die anatomischen Anpassungselemente (11, 22) auf den Platten (1, 2) mittels einerseits Kontakt von mindestens einem Teil ihrer oberen (111) und ihrer unteren (222) Fläche mit mindestens einem Teil der oberen (1) bzw. der unteren (2) Platte und andererseits Kontakt des männlichen Zwischenmittels (50) mit den weiblichen Befestigungsmitteln (113, 223), die auf den anatomischen Anpassungselementen (11, 22) vorliegen, und mit den weiblichen Befestigungsmitteln (15, 25), die auf den Platten (1, 2) der Prothese vorliegen, befestigt sind.

26. Bandscheibenprothese nach Anspruch 24, **dadurch gekennzeichnet, dass** die männlichen Zwischenmittel (50) über Sicherungsmittel (55) verfügen, die die männlichen Zwischenmittel (50) in der Stellung arretieren, in der sie mit sowohl den weiblichen Befestigungsmitteln (113, 223) der anatomischen Anpassungselemente (11, 22) als auch den weiblichen Befestigungsmitteln (15, 25), die auf den Platten (1, 2) der Prothese vorliegen, zusammenwirken.

27. Bandscheibenprothese nach Anspruch 26, **dadurch gekennzeichnet, dass** die männlichen Zwischenmittel (50) aus einer Schieberplatte (50) in den weiblichen Befestigungsmitteln (113, 223), die auf den anatomischen Anpassungselementen (11, 22) vorliegen, bestehen, um mit den weiblichen Befestigungsmitteln (15, 25), die auf den Platten (1, 2) der Prothese vorliegen, zusammenzuwirken, wobei die Sicherungsmittel (55) der männlichen Zwischenmittel (50) aus mindestens einer Formunregelmäßigkeit (55) bestehen, die auf mindestens einer Seite dieser Schiebeplatte (50) vorliegt und dazu vorgesehen ist, mit mindestens einer Öffnung (550) zusammenwirken, die in den weiblichen Befestigungsmitteln (113, 223) der anatomischen Anpassungselemente und/oder in den weiblichen Befestigungsmitteln (15, 25) der Platten (1, 2) hergestellt sind, wodurch die männlichen Zwischenmittel (50), die von dieser Schiebeplatte (50) gebildet werden, in der Stellung arretiert werden, in der sie mit sowohl den weiblichen Befestigungsmitteln (113, 223) der anatomischen Anpassungselemente (11, 22) als auch den weiblichen Befestigungsmitteln (15, 25), die auf den Platten (1, 2) der Prothese vorliegen, zusammenwirken.

28. Bandscheibenprothese nach Anspruch 26, **dadurch gekennzeichnet, dass** die Sicherungsmittel (55) der männlichen Zwischenmittel (50) aus einer Bohrung in den männlichen Zwischenmitteln (50) und in den weiblichen Befestigungsmitteln (113, 223), die auf den anatomischen Anpassungselementen (11, 22) vorlieben, bestehen, wobei die Bohrung (550) in den weiblichen Befestigungsmitteln (113, 223) der anatomischen Anpassungsmittel (11, 22) dazu vorgesehen sind, einen Sicherungsstift (55) aufzunehmen, der die männlichen Zwischenmittel (50) in der Stellung arretiert, in der sie mit den weiblichen Befestigungsmitteln (15, 25), die auf den Platten (1, 2) der Prothese vorliegen, zusammenwirken.

29. Bandscheibenprothese nach einem der Ansprüche 1 bis 28, **dadurch gekennzeichnet, dass** die Mittelebenen, die die oberen (110, 222) und unteren (111, 220) Flächen mindestens eines der anatomischen Anpassungselemente (11, 22) darstellen, im Wesentlichen parallel sind oder einen spitzen Winkel bilden, wobei die durch einen derartigen Winkel erzielte Neigung ermöglicht, die Gesamtform der Prothese an die Anatomie der Wirbelsäule anzupassen oder möglicherweise Neigungsfehler der Wirbel des Patienten, für den die Prothese vorgesehen ist, zu korrigieren.

30. Bandscheibenprothese nach einem der Ansprüche 1 bis 29, **dadurch gekennzeichnet, dass** gleiche anatomische Anpassungselemente (11, 22) mit unterschiedlichen Platten (1, 2) zusammengefügt werden, deren oberen (10, 24) und unteren (14, 20) Flächen unterschiedliche Winkel erzeugen.

31. Bandscheibenprothese nach einem der Ansprüche 9 bis 30, **dadurch gekennzeichnet, dass** ein Winkel zwischen der oberen Fläche (10) der oberen Platte (1) und der unteren Fläche (20) der unteren Platte (2) entweder durch die Tatsache auferlegt wird, dass die Mittelebenen, die die unteren (20, 14) und oberen (24, 10) Flächen der unteren Platte (2) und/oder der oberen Platte (1) darstellen, einen Winkel erzeugen, oder durch Beschränken von Bewegungen des Kerns (3) dank der Zusammenwirkungsmittel (23, 33) - um eine Stellung, die eine Neigung von mindestens einer der Platten (1, 2) auferlegt.

32. Bandscheibenprothese nach einem der Ansprüche 1 bis 31, **dadurch gekennzeichnet, dass** gleiche Platten (1, 2) mit Kernen (3) unterschiedlicher Dicken und/oder Größen und/oder Formeln zusammengefügt werden.

33. Bandscheibenprothese nach einem der Ansprüche 1 bis 32, **dadurch gekennzeichnet, dass** die anatomischen Anpassungselemente (11, 22) bewegliche Knochenverankerungselemente (60) umfassen, die nach Befestigen der anatomischen Anpassungselemente (11, 22) auf den Platten (1, 2), Einsetzen der Prothese zwischen den Wirbeln und möglicherweise Einstellen der relativen Stellung der unterschiedlichen Elemente (1, 2, 3) der Prothese auf den anatomischen Anpassungselementen (11, 22) befestigt werden.

34. Bandscheibenprothese nach Anspruch 33, **dadurch gekennzeichnet, dass** die beweglichen Knochenverankerungselemente (60) der anatomischen Anpassungselemente (11, 22) aus mindestens einer Platte (61) bestehen, die mit Kerben (62) ausgestattet ist, die so ausgerichtet sind, dass sie der Entfernung der Platte (61) widerstehen, nachdem diese in einen Wirbel eingesetzt wurde, wobei ein entferntes Ende der Platte (61) ein Teil (63) trägt, das so gebogen ist, dass es sich auf sich selbst zusammenlegt, und das dazu vorgesehen ist, als ein Haken in eine Kante (16, 26) einer Öffnung einzugreifen, die in der Nähe des Umfangs der anatomischen Anpassungselemente (11, 22) hergestellt ist.

35. Bandscheibenprothese nach Anspruch 34, **dadurch gekennzeichnet, dass** das Teil (63), das so gebogen ist, dass es sich auf sich selbst zusammenlegt, der gekerbten Platte (61) der beweglichen Knochenverankerungsmittel (60) der anatomischen Anpassungselemente (11, 22) mit einer zweiten Platte (61) verlängert ist, die ebenfalls mit Kerben (62) ausgestattet ist, die so ausgerichtet sind, dass sie der Entfernung der Platte (61) widerstehen, nachdem diese in den Wirbel eingesetzt wurde.

36. Bandscheibenprothese nach einem der Ansprüche 1 bis 32, **dadurch gekennzeichnet, dass** die anatomischen Anpassungselemente (11, 22) bewegliche Knochenverankerungselemente (60) umfassen, die aus mindestens einem Winglet bestehen, das in eine Nut eingesetzt werden soll, die in den angrenzenden Flächen der Wirbel vorgenommen wurde, zwischen die die Prothese implantiert werden soll, wobei das Winglet Kerben (66) umfasst, die so ausgerichtet sind, dass sie der Ausstoßung der Prothese aus ihrer Unterbringung zwischen den Wirbeln widerstehen, wobei ein entferntes Ende des Winglets (60) ein Teil (63) trägt, das so gebogen ist, dass es sich auf sich selbst zusammenliegt, und das dazu vorgesehen ist, als ein Haken in eine Kante (16, 26) einer Öffnung einzugreifen, die in der Nähe des Umfangs der anatomischen Anpassungselemente (11, 22) hergestellt ist.

37. Bandscheibenprothese nach Anspruch 36, **dadurch gekennzeichnet, dass** das Winglet (60) weiterhin einen Stift (64) umfasst, der Abmessungen hat, die so angepasst sind, dass er eng in eine Nut (65) der anatomischen Anpassungselemente (11, 22) und/oder der Platten (1, 2) passt.

## Revendications

1. Prothèse de disque intervertébral comprenant une unité d'une taille donnée comportant au moins trois pièces comprenant un plateau supérieur (1), un plateau inférieur (2) et un noyau (3) qui est mobile au moins par rapport à un plateau, **caractérisée en ce qu'**elle comprend en outre au moins deux éléments d'adaptation anatomiques (11, 22) chacun fixé sur l'un des plateaux supérieur et inférieur (1, 2) via des moyens de fixation (113, 223) et permettant d'ajuster la taille globale de la prothèse, chaque élément d'adaptation (11, 22) ayant, d'une part, une surface (110, 220) en contact avec une surface d'une vertèbre et, d'autre part, une surface (111) dont au moins une partie a une surface en contact avec au moins une partie du plateau (1, 2) sur lequel l'élément d'adaptation anatomique (11, 22) est monté, des éléments d'adaptation anatomiques (11, 22) de différentes tailles étant fixés sur ladite unité d'une taille donnée afin d'adapter la prosthèse à différentes tailles de vertèbres et/ou différents espaces intervertébraux.

2. Prothèse de disque intervertébral selon la revendication 1, **caractérisée en ce que** les éléments d'adaptation anatomiques (11, 22) se composent de couronnes qui entourent les plateaux (1 et 2) et prolongeant respectivement leurs surfaces supérieure (10) et intérieure (20) pour présenter des surfaces de contact de la prothèse avec les vertèbres adjacentes qui sont plus grandes que lorsqu'il n'y a pas d'éléments d'adaptation anatomiques (11, 22).

3. Prothèse de disque intervertébral selon la revendication 1, **caractérisée en ce que** les éléments d'adaptation anatomiques (11, 22) se composent de plateaux, dits anatomiques, qui recouvrent les plateaux (1 et 2) et prolongent respectivement leurs surfaces supérieure (10) et inférieure (20) pour présenter des surfaces de contact de la prothèse avec les vertèbres adjacentes qui sont plus grandes que lorsqu'il n'y a pas d'éléments d'adaptation anatomiques (11, 22).

4. Prothèse de disque intervertébral selon l'une quelconque des revendications 1 à 3, **caractérisée en ce que** les éléments d'adaptation anatomiques (11, 22) prolongent symétriquement les surfaces supérieure (10) et inférieur (20) des plateaux, respectivement, supérieure (1) et inférieure (2) pour présenter un prolongement equivalent de ces surfaces (10, 20) sur les différents bords antérieur, postérieur et latéraux des plateaux (1, 2).

5. Prothèse de disque intervertébral selon l'une quelconque des revendications 1 à 3, **caractérisée en ce que** les éléments d'adaptation anatomiques (11, 22) prolongent asymétriquement les surfaces supérieure (10) et inférieur (20) des plateaux, respectivement, supérieure (1) et inférieure (2) pour présenter un plus grand prolongement de ces surfaces (10, 20) sur au moins l'un des bords antérieurs, postérieurs et latéraux des plateaux (1, 2) que sur les autres bords.

6. Prothèse de disque intervertébral selon l'une quelconque des revendications 1 à 5, **caractérisée en ce qu'**une surface supérieure (30) du noyau (3) est en contact avec au moins une partie d'une surface inférieure (14) du plateau supérieur (1) et une surface inférieure (34) du noyau (3) est en contact avec au moins une partie d'une surface supérieure (24) du plateau inférieur (2).

7. Prothèse de disque intervertébral selon l'une quelconque des revendications 1 à 6, **caractérisée en ce qu'**au moins une partie (140) d'une surface (14, 24) d'au moins un plateau (1, 2) est concave et complémentaire d'une surface convexe (30) du noyau (3) avec laquelle elle est en contact.

8. Prothèse de disque intervertébral selon l'une quelconque des revendications 1 à 7, **caractérisée en ce qu'**au moins une partie d'une surface (14, 24) d'au moins un plateau (1, 2) est plane et complémentaire d'une surface plane (34) du noyau (3) avec laquelle elle est en contact.

9. Prothèse de disque intervertébral selon l'une quelconque des revendications 1 à 8, **caractérisée en ce que** des moyens de coopération mâle et femelle (23, 33) situés à proximité des bords d'au moins une plateau (1, 2) et du noyau (3) limitent les mouvements de translation du noyau (3) par rapport à ce plateaux (1, 2), selon un axe sensiblement parallèle à ce plateau (1, 2), et limitent ou suppriment les mouvements de rotation du noyau (3) par rapport à ce plateau (1, 2), autour d'un axe sensiblement perpendiculaire à ce plateau (1, 2).

10. Prothèse de disque intervertébral selon la revendication 9, **caractérisée en ce que** les dimensions de chaque moyen de coopération mâle (33) sont généralement inférieures à celles de chaque moyen de coopération femelle (23) pour permettre un léger débattement entre le noyau (3) et le plateau (1, 2) équipé de ces moyens de coopération.

11. Prothèse de disque intervertébral selon la revendication 9, **caractérisée en ce que** les dimensions de chaque moyen de coopération mâle (33) sont sensiblement les mêmes que celles de chaque moyen de coopération femelle (23) afin d'empêcher tout débattement entre le noyau (3) et le plateau (1, 2) équipé de ces moyens de coopération.

12. Prothèse de disque intervertébral selon l'une quelconque des revendications 9 à 11, **caractérisée en ce que** les moyens de coopération (23) du plateau (1, 2) sont des moyens de coopération femelles coopérant avec des moyens de coopération mâles (33) du noyau (3).

13. Prothèse de disque intervertébral selon la revendication 12, **caractérisée en ce que** les moyens de coopération mâles (33) du noyau (3) sont deux blocs situés sur les deux bords latéraux du noyau (3) et les moyens de coopération femelle (23) du plateau (1, 2) sont quatre parois situées, par paires, sur chacun des deux bords latéraux de ce plateau (1, 2).

14. Prothèse de disque intervertébral selon l'une quelconque des revendications 1 à 13, **caractérisée en ce que** les moyens de fixation (113, 223, 15, 25) des éléments d'adaptation anatomiques (11, 22) sur les plateaux (1, 2) de la prothèse sont réversibles et permettent de changer les éléments d'adaptation anatomiques (11, 22) fixés d'une marnière amovible sur les plateaux (1, 2) de la prothèse.

15. Prothèse de disque intervertébral selon l'une quelconque des revendications 1 à 14, **caractérisée en ce que** les moyens de fixation (113, 223, 15, 25) des éléments d'adaptation anatomique (11, 22) sur les plateaux (1, 2) se composent de moyens de fixation (113, 223) présents sur les éléments d'adaptation anatomiques (11, 22) et complémentaires de moyens de fixation (15, 25) présents sur les plateaux (1, 2) de la prothèse.

16. Prothèse de disque intervertébral selon la revendication 15, **caractérisée en ce que** les éléments d'adaptation anatomiques (11, 22) sont fixés sur les plateaux (1, 2), d'une part, via le contact avec au moins une partie de leurs surfaces (111 et 222) qui font face à au moins une partie des plateaux (1, 2) et, d'autre part, le contact de leurs moyens de fixation (113, 223) avec les moyens de fixation (15, 25) complémentaires présents sur les plateaux (1, 2) de la prothèse.

17. Prothèse de disque intervertébral selon la revendication 15 ou 16, **caractérisée en ce que** les moyens de fixation (113, 223, 15, 25) des éléments d'adaptation anatomiques (11, 22) sur les plateaux (1, 2) se composent de moyens de fixation mâles (113, 223) présents sur les éléments d'adaptation anatomiques (11, 22) et coopérant avec des moyens de fixation femelles (15, 25) présents sur les plateaux (1, 2) de la prothèse ou inversement.

18. Prothèse de disque intervertébral selon la revendication 17, **caractérisée en ce que** les moyens de fixation femelles (15, 25) présents sur les plateaux (1, 2) de la prothèse se composent de surfaces planes (15, 25) présentes sur les bords des plateaux (1, 2) de la prothèse.

19. Prothèse de disque intervertébral selon la ; revendication 17, dans laquelle les moyens de fixation femelles (15, 25) présents sur les plateaux (1, 2) de la prothèse se composent d'évidements (15, 25) réalisés dans les bords des plateaux (1, 2) de la prothèse.

20. Prothèse de disque intervertébral selon la revendication 17, **caractérisée en ce que** les moyens de fixation femelles (15, 25) présents sur les plateaux (1, 2) de la prothèse se composent d'évidements réalisés dans les bords des moyens de coopération femelles (23) des plateaux (1, 2) de la prothèse.

21. Prothèse de disque intervertébral selon la revendication 17, **caractérisée en ce que** les moyens de fixation femelles (15, 25) présents sur les plateau (1, 2) de la prothèse se composent de surfaces plane (15) présentes sur les bords de l'un des plateaux (1) et d'évidements (25) réalisés dans les moyens de coopération femelles (23) des bords de l'autre plateau (2) de la prothèse.

22. Prothèse de disque intervertébral selon la revendication 17, **caractérisée en ce que** les moyens de fixation femelles (15, 25) présents sur au moins l'un des plateaux (1, 2) de la prothèse se composent de surfaces planes (15, 25) présentes sur au moins un premier bord de l'un des plateaux (1, 2) et d'évidements (15, 25) réalisés dans au moins un second bord du plateau (1, 2) de la prothèse, le second bord faisant géométriquement face au premier bord du plateau (1, 2).

23. Prothèse de disque intervertébral selon l'une quelconque des revendications 17 à 22, **caractérisée en ce qu'**au moins l'un des moyens de fixation femelles (15, 25) présents sur les plateaux (1, 2) de la prothèse comprend au moins une encoche (55) permettant de bloquer les moyens de fixation mâles (113, 223) des éléments d'adaptation anatomiques (11, 22) sur ces moyens de fixation femelles (15, 25).

24. Prothèse de disque intervertébral selon l'une quelconque des revendications 1 à 16, **caractérisée en ce que** les moyens de fixation (113, 223, 15, 25) des éléments d'adaptation anatomiques (11, 22) sur les plateaux (1, 2) se composent de moyens de fixation femelles (113, 223) présents sur les éléments d'adaptation anatomiques (11, 22) et coopérant avec des moyens intermédiaires mâles (50) qui coopèrent également avec des moyens de fixation femelles (15, 25) présents sur les plateaux (1, 2) de la prothèse.

25. Prothèse de disque intervertébral selon la revendication 24, **caractérisée en ce que** les éléments d'adaptation anatomiques (11, 22) sont fixés sur les plateaux (1, 2), d'une part, via le contact d'au moins une partie de leurs surfaces supérieure (111) et inférieure (222) avec au moins une partie des plateaux, respectivement supérieur (1) et inférieur (2) et, d'autre part, le contact des moyens intermédiaires mâles (50) avec les moyens de fixation femelles (113, 223) présents sur les éléments d'adaptation anatomiques (11, 22) et avec les moyens de fixation femelles (15, 25) présents sur les plateaux (1, 2) de la prothèse.

26. Prothèse de disque intervertébral selon la revendication 24, **caractérisée en ce que** les moyens intermédiaires mâles (50) possèdent des moyens de fixation (55) bloquant les moyens intermédiaires mâles (50) dans la position dans laquelle ils coopèrent à la fois avec les moyens de fixation femelles (113, 223) des éléments d'adaptation anatomiques (11, 22) et avec les moyens de fixation femelles (15, 25) présents sur les plateaux (1, 2) de la prothèse.

27. Prothèse de disque intervertébral selon la revendication 26, **caractérisée en ce que** les moyens intermédiaires mâles (50) se composent d'une plaque coulissante (50) dans les moyens de fixation femelles (113, 223) présents sur les éléments d'adaptation anatomiques (11, 22) pour coopérer avec les moyens de fixation femelles (15, 25) présents sur les plateaux (1, 2) de la prothèse, les moyens de fixation (55) des moyens intermédiaires mâles (50) se composant d'au moins une irrégularité de forme (55) présente sur au moins un côté de cette plaque coulissante (50) et prévue pour coopérer avec au moins une ouverture (550) réalisée dans les moyens de fixation femelles (113, 223) des éléments d'adaptation anatomiques (11, 22) et/ou dans les moyens de fixation femelles (15, 25) des plateaux (1, 2), bloquant ainsi les moyens intermédiaires mâles formés par cette plaque coulissante (50) dans la position dans laquelle ils coopèrent à la fois avec les moyens de fixation femelles (113, 223) des éléments d'adaptation anatomiques (11, 22) et avec les moyens de fixation femelles (15, 25) présents sur les plateaux (1, 2) de la prothèse.

28. Prothèse de disque intervertébral selon la revendication 26, **caractérisée en ce que** les moyens de fixation (55) des moyens intermédiaires mâles (50) se composent d'un alésage dans les moyens intermédiaires mâles (50) et dans les moyens de fixation femelles (113, 223) présents sur les éléments d'adaptation anatomiques (11, 22), l'alésage (550) dans les moyens de fixation femelles (113, 223) des éléments d'adaptation anatomiques (11, 22) étant prévu pour recevoir une broche de fixation (55) bloquant les moyens intermédiaires mâles (50) dans la position dans laquelle ils coopèrent avec les moyens de fixation femelles (15, 25) présents sur les plateaux (1, 2) de la prothèse.

29. Prothèse de disque intervertébral selon l'une quelconque des revendications 1 à 28, **caractérisée en ce que** les planes médians représentant les surfaces supérieure (110, 222) et inférieure (111, 220) d'au moins l'un des éléments d'adaptation anatomiques (11, 22) sont sensiblement parallèles ou forment un angle aigu, l'inclinaison obtenue avec un tel angle permettant d'adapter la forme globale de la prothèse à l'anatomie de la colonne vertébrale ou de corriger éventuellement des défauts d'inclinaison des vertèbres du patient pour lequel la prothèse est prévue.

30. Prothèse de disque intervertébral selon l'une quelconque des revendications 1 à 29, **caractérisée en ce que** les mêmes éléments d'adaptation anatomiques (11, 22) sont assemblés avec différentes plateaux (1, 2) dont les surfaces supérieure (10, 24) et inférieure (14, 20) créent des angles différents.

31. Prothèse de disque intervertébral selon l'une quelconque des revendications 9 à 30, **caractérisée en ce qu'**un angle entre la surface supérieure (10) du plateau supérieur (1) et la surface inférieure (20) du plateau inférieur (2) est imposé par le fait que les plans médians représentant les surfaces inférieur (20, 14) et supérieure (24, 10) du plateaux inférieur (2) et/ou du plateau supérieur (1) créent un angle, ou en limitant, grâce aux moyens de coopération (23, 33), les mouvements du noyau (3) autour d'une position imposant une inclinaison d'au moins l'un des plateaux (1, 2).

32. Prothèse de disque intervertébral selon l'une quelconque des revendications 1 à 31, **caractérisée en ce que** les mêmes plateaux (1, 2) sont assemblées avec des noyaux (3) de différentes épaisseurs et/ou tailles et/ou formes.

33. Prothèse de disque intervertébral selon l'une quelconque des revendications 1 à 32, **caractérisée en ce que** les éléments d'adaptation anatomiques (11, 22) comprennent des moyens d'ancrage osseux mobiles (60) qui sont fixés sur les éléments d'adaptation anatomiques (11, 22) après avoir fixé les éléments d'adaptation anatomiques (11, 22) sur les plateaux (1, 2), en insérant la prothèse entre les vertèbres et en ajustant éventuellement la position relative des différentes éléments (1, 2, 3) de la prothèse.

34. Prothèse de disque intervertébral selon la revendication 33, **caractérisée en ce que** les éléments d'ancrage osseux mobiles (60) des éléments d'adaptation anatomiques (11, 22) se composent d'au moins une plaque (61) équipée d'encoches (62) orientées pour résister au retrait de la plaque (61) une fois qu'elle a été insérée dans une vertèbre, une extrémité de la plaque (61) portant une partie (63) incurvée pour se replier sur elle-même et prévue pour être verrouillée comme un crochet sur un bord (16, 26) d'une ouverture réalisée à proximité de la périphérie des éléments d'adaptation anatomiques (11, 22).

35. Prothèse de disque intervertébral selon la revendication 34, **caractérisée en ce que** la partie (63), incurvée pour se replier sur elle-même, de la plaque crantée (61) des moyens d'ancrage osseux mobiles (60) des éléments d'adaptation anatomiques (11, 22) se prolonge par une seconde plaque (61) également équipée d'encoches (62) orientées pour résister au retrait de la plaque (61) une fois qu'elle a été insérée dans la vertèbre.

36. Prothèse de disque intervertébral selon l'une quelconque des revendications 1 à 32, **caractérisée en ce que** les éléments d'adaptation anatomiques (11, 22) comprennent des éléments d'ancrage osseux mobiles (60) se composant d'au moins une ailette à insérer dans une rainure réalisée dans les surfaces adjacentes des vertèbres entre lesquelles la prothèse doit être implantée, ladite ailette comprenant des encoches (66) orientées pour résister à l'éjection de la prothèse à l'extérieur de son logement entre les vertèbres, une extrémité de l'ailette (60) supportant une partie (63) incurvée pour se replier sur elle-même et prévue pour être bloquée comme un crochet sur un bord (16, 26) d'une ouverture réalisée à proximité de la périphérie des éléments d'adaptation anatomiques (11, 22).

37. Prothèse de disque intervertébral selon la revendication 36, **caractérisée en ce que** l'ailette (60) comprend en outre une broche (64) ayant des dimensions adaptées de sorte qu'elle s'ajuste étroitement dans une rainure (65) des éléments d'adaptation anatomiques (11, 22) et/ou des plateaux (1, 2).
